# EUROPEAN PATENT SPECIFICATION

(11) **EP 1 751 553 B1**
(45) Date of publication and mention of the grant of the patent: **08.10.2008**
(21) Application number: 05759896.3
(22) Date of filing: 24.05.2005
(51) Int. Cl.: G01N 33/68, A61K 31/155, C07C 281/18, C07C 243/24, A61K 31/16

(54) **IDENTIFICATION OF ANTI-PRION DRUGS BY HIGH-THROUGHPUT SCREENING BASED ON SIFT**
IDENTIZIFIERUNG VON ANTIPRIONWIRKSTOFFEN MITTELS SCREENING MIT HOHEM DURCHSATZ AUF SIFT-BASIS
IDENTIFICATION SYSTEMATIQUE DE NOUVEAUX MEDICAMENTS ANTI-PRIONS PAR CRIBLAGE A HAUT RENDEMENT FONDE SUR UN BALAYAGE DE CIBLES INTENSEMENT FLUORESCENTES (SIFT)

(30) Priority: 24.05.2004 EP 04012254
(43) Date of publication of application: 14.02.2007
(73) Proprietor: Ludwig-Maximilians-Universität München, 80802 München (DE); Max-Planck-Gesellschaft zur Förderung der Wissenschaften e.V., Berlin (DE)
(72) Inventor: BERTSCH, Uwe, 86937 Scheuring (DE); GIESE, Armin, 84177 München (DE); KRETZSCHMAR, Hans, 82515 Wolfratshausen (DE); TAVAN, Paul, 80805 München (DE); HIRSCHBERGER, Thomas, 80339 München (DE); BIESCHKE, Jan, San Diego, CA 92109 (US); WEBER, Petra, 85221 Dachau (DE); WINKLHOFER, Konstanze, F., 81247 München (DE); TATZELT, Jörg, 81247 München (DE); HARTL, F., Ulrich, 82288 Kottgeisering (DE); WÜNSCH, Gerda, 82515 Wolfratshausen (DE); HÖGEN, Tobias, Johannes, 82216 Maisach (DE)
(74) Representative: Vossius & Partner
(86) International application number: PCT/EP2005/005614
(87) International publication number: WO 2005/116640

(56) References cited:
- WO-A-01/25192
- WO-A-20/05031000
- BIESCHKE J G ET AL: "SINGLE MOLECULE FLUORESCENCE DETECTION AND CHARACTERIZATION OF AMYLOID PROTEIN AGGREGATION" BIOPHYSICAL JOURNAL, NEW YORK, US, US, vol. 84, no. 2, PART 2, February 2003 (2003-02), page 313A, XP009033222 ISSN: 0006-3495
- BIESCHKE J ET AL: "ULTRASENSITIVE DETECTION OF PATHOLOGICAL PRION PROTEIN AGGREGATES BY DUAL-COLOR SCANNING FOR INTENSELY FLUORESCENT TARGETS" PROCEEDINGS OF THE NATIONAL ACADEMY OF SCIENCES OF USA, NATIONAL ACADEMY OF SCIENCE, WASHINGTON, DC, US, vol. 97, no. 10, 9 May 2000 (2000-05-09), pages 5468-5473, XP000999285 ISSN: 0027-8424
- BRYANT HENRY U ET AL: "A novel class of 5-HT-2A receptor antagonists: Aryl aminoguanidines" LIFE SCIENCES, vol. 59, no. 15, 1996, pages 1259-1268, XP002360667 ISSN: 0024-3205
- GUNES H SEMIH: "Synthesis and antimicrobial screening of some N-(3,4-dimethoxyphenetyl)-N-substituted benzylideneamine derivatives" JOURNAL OF FACULTY OF PHARMACY OF GAZI UNIVERSITY, vol. 18, no. 2, 2001, pages 89-97, XP008057517 ISSN: 1015-9592
- JENEY E ET AL: "[The antibicrobical action of new aminoguanidones]" ZENTRALBLATT FUR BAKTERIOLOGIE, PARASITENKUNDE, INFEKTIONSKRANKHEITEN UND HYGIENE. 1. ABT. MEDIZINISCH-HYGIENISCHE BAKTERIOLOGIE, VIRUSFORSCHUNG UND PARASITOLOGIE. ORIGINALE. MAY 1969, vol. 210, no. 1, May 1969 (1969-05), pages 107-114, XP008057514 ISSN: 0372-8110
- JENEY E ET AL: "[Aminoguanidones from alkyloxy-acetophenones as locally applicable bacteriostatics against polyresistant staphylococci]" ZENTRALBLATT FUR BAKTERIOLOGIE, PARASITENKUNDE, INFEKTIONSKRANKHEITEN UND HYGIENE. 1. ABT. MEDIZINISCH-HYGIENISCHE BAKTERIOLOGIE, VIRUSFORSCHUNG UND PARASITOLOGIE. ORIGINALE. JAN 1966, vol. 199, no. 1, January 1966 (1966-01), pages 109-112, XP008057513 ISSN: 0372-8110
- GIESE A ET AL: "Putting prions into focus: Application of single molecule detection to the diagnosis of prion diseases" ARCHIVES OF VIROLOGY SUPPLEMENT, no. 16, 2000, pages 161-171, XP001000222 ISSN: 0939-1983
- BERTSCH UWE ET AL: "Systematic identification of antiprion drugs by high-throughput screening based on scanning for intensely fluorescent targets" JOURNAL OF VIROLOGY, vol. 79, no. 12, June 2005 (2005-06), pages 7785-7791, XP002360668 ISSN: 0022-538X

## Description

The present invention relates to a method of identifying a compound for inhibiting aggregation of proteins involved in diseases linked to protein aggregation and/or neurodegenerative diseases, comprising the steps of: (a) bringing into contact a labeled monomeric protein and a differently labeled aggregate of said protein in the (1) presence and (2) absence of a candidate inhibitor of aggregation, (b) determining the amount of co-localized labels, representing the extent of binding of the monomeric proteins to the aggregates of said protein; and (c) comparing the result obtained in the presence and absence of said compound, wherein a decrease of co-localized labels in the presence of said compound is indicative of the compound's ability to inhibit aggregation of said protein. Moreover, the present invention relates to a pharmaceutical composition containing said inhibitor of aggregation as well as to a kit.

Several documents are cited throughout the text of this specification.

A large number of neurological and neurodegenerative disease are known, many of which are presently not curable. These diseases comprise medical conditions such as Parkinson's disease, Chorea. Huntington, Hallervorder-Spatz disease, Alzheimer's disease, senile dementia, Creutzfeldt-Jakob disease, artheriosclerotic dementia, cerebral thrombangitis obliterans and many others.

Prion diseases include diseases such as Creutzfeldt-Jakob disease (CJD), scrapie and bovine spongiform encephalopathy (BSE). Pathologically the prion diseases are characterized by a spongiform degeneration of the brain. They are caused by an unconventional infectious agent which consists primarily of the missfolded, aggregated, beta-sheet rich PrPSc isoform of the membrane glycoprotein PrPC.

Prion diseases have caused a major concern in regard to public health due to the emergence of BSE. Scientific evidence suggests that BSE has been transmitted to humans causing a new variant of Creutzfeldt-Jakob disease (vCJD) (Will et al 1996, Bruce et al. 1997). It is unknown how many people are currently incubating the disease and will be affected by vCJD in the future. The available evidence does not exclude an impending epidemic affecting a large number of patients (Andrews et al. 2000). This heightens the need to develop effective therapeutics in addition to implementing measures preventing further spread of the disease. In addition recent evidence suggest that secondary transmission by blood transfusion may occur (LLewelyn et al.,2004).

The central event in the pathogenesis of prion diseases is the conversion of the cellular prion protein PrPC into the pathological PrPSc isoform. A hallmark of pathogenesis is the formation of large protein aggregates formed by aggregating PrPSc. The available evidence suggests that PrPSc acts both as a template for this conversion and as a neurotoxic agent causing neuronal dysfunction and cell death (Prusiner 1998, Giese and Kretzschmar 2001). Therefore, the most promising therapeutic approach for prion diseases is to interfere with PrPSc amplification. Evidence derived from cell culture and in vivo studies suggests that once formation of PrPSc is inhibited, clearance of PrPSc can take place (Mallucci 2003). Thus, this therapeutic strategy could also be effective late in the incubation period and even after manifestation of clinical signs of disease, which is essential to be of use in addressing human prion disease.

There are a number of compounds which have been shown to be effective in interfering with PrPSc amplification in vitro such as Congo Red, porphyrins/phthalocyanines, Cp-60, beta-sheet breaker peptides and variants of PrP (Caughey et al 1998, Chabry et al. 1998, Demaimay et al. 2000, Horiuchi et al. 2000, Perrier et al. 2000, Rudyk et al. 2000, Soto et al. 2000). However, none of these compounds has so far successfully been used for disease treatment or as lead compounds for developing compounds with increased therapeutic potency and pharmacological properties. Moreover, few in vitro assays that can be used for high-throughput screening of large compound libraries for potential anti-prion drugs have been established: only two different approaches towards a systematic screening for anti-prion compounds have been proposed. Both of these two screening assays, the one being yeast-based (Bach et al. 2003) and the other using infected ScN2a cell cultures (Kocisko et al. 2004, Kocisko et al. 2003). allowed the screening of libraries containing only 2500 and 2000 compounds, respectively. However, the assays turned out to be quite time-consuming.

Bieschke J.G. et al. (Biophys.J. (2003), vol.84, page 313A) shows a method of identifying a potential therapeutic compound for interfering with the aggregation of proteins involved in diseases linked to protein aggregation and/or neurodegenerative diseases. The present subject-matter differs therefrom in that a special group of potential inhibitors according to formula (I) is screened for inhibiting protein aggregation. Some similar compounds are known in the prior art as antimicrobial agents (Günes, H.S., J.Fac.Pharm.Gazi (2001), Vol.18, no.2, pages 89-97).

Hence, there is an urgent need for identifying novel compounds suitable for the treatment of prion diseases. A similar need also exists with respect to other diseases linked to aggregating proteins.

Thus, the technical problem underlying the present invention was to provide means and methods for treating prion diseases and other diseases characterized by aggregating proteins.

The solution to this technical problem is achieved by providing the embodiments characterized in the claims.

Accordingly, the present invention relates to a method of identifying a compound for inhibiting aggregation of proteins involved in diseases linked to protein aggregation and/or neurodegenerative diseases, comprising the steps of: (a) bringing into contact a labeled monomeric protein and a differently labeled aggregate of said protein in the (1) presence and (2) absence of a candidate inhibitor of aggregation which is selected from the group represented by formula (I) 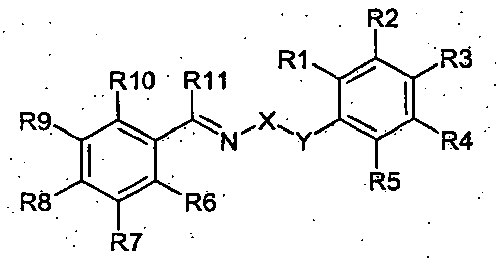 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R12 (if X is CH-R12), R13 (if X is N-R13) and R14 (if Y is CH-R14) are independently selected from hydrogen, alkyl, cycloalkyl heterocycloalkyl, or hydroxyalkyl, each of which is optionally substituted

(b) determining the amount of co-localized labels, representing the extent of binding of the monomeric proteins to the aggregates of said protein; and (c) comparing the result obtained in the presence and absence of said compound, wherein a decrease of co-localized labels in the presence of said compound is indicative of the compound's ability to inhibit aggregation of said protein.

Also described is a method of identifying a compound for inhibiting aggregation of proteins involved in diseases linked to protein aggregation and/or neurodegenerative diseases, comprising the steps of: (a) bringing into contact a labeled monomeric protein and a differently labeled aggregate of said protein in the (1) presence and (2) absence of a candidate inhibitor of aggregation which is selected from the group represented by formula (II) 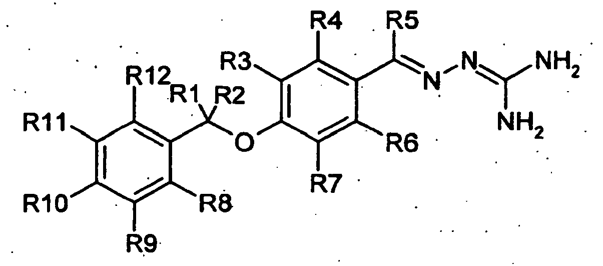 wherein in formula (II):
R1 and R2 are independently selected from hydrogen, alkyl, cycloalkyl heterocycloalkyl, or hydroxyalkyl, each of which is optionally substitute;
R3, R4, R5, R6, R7, R8, R9, R10, R11 and R12 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
(b) determining the amount of co-localized labels, representing the extent of binding of the monomeric proteins to the aggregates of said protein; and (c) comparing the result obtained in the presence and absence of said compound, wherein a decrease of co-localized labels in the presence of said compound is indicative of the compound's ability to inhibit aggregation of said protein.

As used herein, "aggregation" means the formation of multimeric, complexes of typically one or more types of proteins, which may be accompanied by the integration of additional biomolecules, like carbohydrates, nucleic acids and lipids, into the complexes.

The term "monomeric protein" means a molecular unit composed of one single, (poly)peptide chain with a three-dimensional conformation specific for each particular protein which is preferably soluble in aqueous solutions up to typically nanomolar, micromolar or milimolar concentrations and may be modified by covalent linkage of one or more carbohydrates, carbohydrate derivatives, lipids, phosphate, sulfate, fatty acids, and nucleotides to individual amino acids in the chain. Preferably, said modification is a phosphorylation, glycosylation, proteolytic processing, glycation, oxidation, and nitration. As used throughout the present invention, the term "protein" also refers to (poly)peptides.

The term "aggregated protein" means non-covalently linked complexes of one or more types of "monomeric protein(s) or polypeptide(s)", as defined above, which are characterized by an altered three-dimensional conformation of the complexed protein units with respect to the monomeric protein units and a typically low solubility of the complexes in aqueous solutions.

The term "compound for inhibiting protein aggregation" refers to a molecular or macromolecular compound which is capable of preventing the formation of protein aggregates and/or which is capable of disintegrating or breaking down existing protein aggregates. Said compound may be, for example, a "small molecular compound" or a (poly)peptide or a derivative thereof.

The term "small molecule" or "small molecular compound" refers to a compound having a relative molecular weight of not more than 1000 D and preferably of not more than 500 D. It can be of organic or inorganic nature. A large number of small molecule libraries, which are commercially available, are known in the art, like the DiverSet library from Chembridge. Thus, for example, said compound may be any of the compounds contained in such a library or a modified compound derived from a compound contained in such a library. Preferably, such a compound binds the monomeric and/or aggregated protein with sufficient specificity, wherein sufficient specificity means preferably a dissociation constant (Kd) of less than 500nM, more preferable less than 200nM, still more preferable less than 50nM, even more preferable less than 10nM and most preferable less than 1 nM.

(Poly)peptide-based or peptide-based compounds may be selected by using, for example, any of the phage libraries and the well established phage display techniques. Preferably, the (poly)peptide-based compound inhibiting protein aggregation may be an antibody.

Said compounds can be derived from preexisting compounds (e.g. compounds from a library) by derivatization. Preferably, such compounds are designed by computer modeling, wherein computer modeling means using virtual-screening tools for the search of compounds that bind to the monomeric or the aggregated form of the protein or both. Generally, these methods rely on the three-dimensional structure of proteins, preferably of proteins crystallized together with a substrate. More preferably, the substrate is replaced with a candidate modulator or inhibitor.

The term "proteins involved in diseases linked to protein aggregation and/or neurodegenerative diseases" as used herein, refers to those diseases which are characterized by the presence of aggregated proteins. Such aggregated proteins may form deposits in specific tissue, more preferably in nerve tissue or tissue of the brain. The extent of aggregation depends on the particular disease.

The term "labeled ... protein" refers to a protein to which a label is attached. Said label may be attached directly or indirectly. Indirect labeling particularly refers to labeled (poly)peptides, more particularly labeled antibodies. Attachment of the label can be performed by a number of techniques known to the person skilled in the art and described in standard textbooks (see for example Harlow and Lane "Antibodies, A Laboratory Manual", CSH Press, Cold Spring Harbor, 1998).

The term "differently labeled protein" means that different labels are attached to the aggregated and the monomeric isoform of the protein. A typical example is the attachment of "FITC" to the aggregated protein and of "Texas red" to the monomeric protein. Since these labels are detectable at different wavelength of light, it is possible to determine the amount and/or the location of the isoforms of the protein. More particularly, the use of different labels allows to quantify the presence of co-localized labels, i.e. of labels which are found in close proximity to each other.

"Determining the amount of co-localized labels" may be performed, e.g. by separately measuring (i.e. wavelength-specific) the number of single photons of at least two different wavelengths coming from the same small volume element of typically less than 1 femtoliter of a sample within a very short time period of typically less than 100 µs followed by the computerized comparison of the respective photon numbers, which may be represented graphically in a multidimensional histogram with one axis for the number of photons of one particular wavelength. In the case of two wavelengths the photon numbers of a particular time period may thus be represented as single dots in a two dimensional fluorescence intensity histogram.

The term "comparing the result obtained in the presence and absence of said compound" means assessing the effect, of the compound on the formation and/or amount of protein aggregates. As used herein, a decrease of co-localized labels of more than 10%, more preferably of more than 25%, even more preferably of more than 50% and most preferably of more than 95%, in the presence of a candidate inhibitor compound of aggregation, is indicative of the compounds ability to inhibit protein aggregation. The term "absence of said compound" means that no inhibitor or candidate inhibitor is or has been added to the aggregating protein. In particular cases it may be useful to add negative controls, i.e. compounds which have no effect on protein aggregation. The term "absence of said compound" also refers to these cases. Likewise, any of the compounds of the present application which inhibit protein aggregation may be used as positive controls in assays for identifying novel inhibitor compounds. It is apparent that the term "presence" also refers to quantity. For apparent reasons, the compounds referred to in the present invention have different effective concentrations. Preferably effective concentrations are less than 100 µM, more preferably below 10 µM and even more preferably below 1 µM.

The method of the present invention is particularly useful for identifying novel compounds capable of interfering with protein aggregation. It allows screening of large libraries of compounds and permits identifying inhibiting compounds with high fidelity. In one aspect of the present invention, the method is based on fluorescence correlation spectroscopy. In recent years fluorescence correlation spectroscopy (FCS) has been recognized as a method that allows highly sensitive analysis of protein aggregation in neurodegenerative diseases such as prion diseases at the molecular level (Bieschke and Schwille 1997, Bieschke et al. 2000, Giese et al. 2000, Post et al. 1998). Moreover, FCS lends itself to miniaturization and automation and has become an established method for high-throughput screening in the pharmaceutical industry (Koltermann et al. 1998). Fluorescence correlation spectroscopy (FCS) in its current confocal form analyses the signal fluctuations caused by the diffusion of single fluorescently labeled molecules through an open volume element defined by the beam of an excitation laser focused through a high aperture microscope objective and confocally imaged on a single photon counting detector (Schwille et al. 1997). In its most preferred embodiment, the method of the present invention is based on this technology. This method is suited for high-throughput screening based on the inhibition of, for example, PrPC binding to aggregate of PrPSc.

Applying the teaching disclosed in the, present invention, the inventors have demonstrated that an assay system based on the SIFT technique can be used for the in vitro screening of large libraries of synthetic compounds for inhibitors of the aggregation processes accompanying neurodegenerative diseases and in particular prion diseases at the molecular level. Such inhibitors bear the potential of being novel therapeutics for these diseases. This novel assay system surpasses by far all assay systems in use for the search of novel anti-prion drugs with respect to the degree of automation, the speed of measurement (75 seconds per sample), the amount of chemical compounds (only 200 picomoles per primary assay) as well as infectious agent (only the equivalent 0.2 mg of brain from a CJD-case per assay) needed. Only these relatively low requirements of resources and time allow the screening of such high numbers of compounds. Furthermore, the mapping of all screening data onto a centralized data base and their automated analysis allow efficient evaluation and analysis of structure-activity relationships. Traditional cell culture screening procedures, which were included for evaluation purposes of hits, were used once before on a library of 2000 substances (Kocisko 2003) at an enormous effort. In contrast, as shown herein it is possible to optimise the primary screening to a throughput of 500-1000 compounds per day within an university setting, employing one technician and a liquid handling robot as well as one Insight^{™} Reader FCS-instrument.

This new assay system for the detection of inhibitors of protein aggregation should also be adaptable to the search of new therapeutics for other neurodegenerative diseases that are linked to aggregation of specific proteins such as Alzheimer's disease and Parkinson'disease. Moreover, it should be possible to search for potential therapeutics for all diseases, where multimer formation plays a crucial role in pathogenesis irrespective of the chemical nature of their components.

In a preferred embodiment of the present invention, said labels are fluorescent labels. Preferably, the label is selected from the group consisting of fluorochromes, e.g. fluorescein isothiocyanate (FITC), rhodamine, Texas Red, Alexa 488, Alexa 647, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where e.g. the protein or (poly)peptide or compound of the present invention is conjugated to biotin, haptens, etc. having a high affinity binding partner, e.g. avidin, specific antibodies, etc., where the binding partner is conjugated to a detectable label.

In another preferred embodiment of the present invention, said label is attached to an antibody or a fragment of an antibody specifically bound to said protein.

The term "specific binding" of antibodies may be described, for example, in terms of their cross-reactivity. Preferably, "antibody specifically bound to ..." refers to antibodies that do not bind (poly)peptides with less than 98%, less than 95%, less than 90%, less than 85%, less than 80%, less than 75%, less than 70% and less than 65% identity (as calculated using methods known in the art) to a (poly)peptide encoded by the aggregating protein. Antibodies may, however, also be described or specified in terms of their binding affinity. Preferred binding affinities include those with a dissociation constant or Kd less than 5X10⁻⁶M, 10⁻⁶M, 5X10⁻⁷M, 10⁻⁷M, 5X10⁻⁸M, 10⁻⁸M, 5X10⁻⁹M, 10⁻⁹M, 5X10⁻¹⁰M, 10⁻¹⁰M, 5X10⁻¹¹M, 10⁻¹¹M, 5X10⁻¹²M, 10⁻¹²M, 5X10⁻¹³M, 10⁻¹³M, 5X10⁻¹⁴M, 10⁻¹⁴M, 5X10⁻¹⁵M, and 10⁻¹⁵M.

The term "antibody" refers to polyclonal, monoclonal, chimeric, single chain, single chain Fv, antibody, or Fab fragment. The skilled person knows that in many cases antibodies can be replaced with other specifically binding compounds such as peptides exposed on the surface of phages (phage display) or with isolated (poly)peptides. The antibody or (poly)peptides may be unlabeled or labeled with any of the labels described in the present invention. The term "(poly)peptide" refers alternatively to peptide or to (poly)peptides. Peptides conventionally are covalently linked amino acids of up to 30 residues, whereas polypeptides (also referred to as "proteins") comprise 31 and more amino acid residues. Preferably, antibodies are obtainable from human, mouse, rat, goat or rabbit.

In a more preferred embodiment of the present invention, said antibody is capable of discriminating between the aggregated and monomeric protein. The term "capable of discriminating" refers to an antibody which is specific to either the monomeric or the aggregated isoform of the protein. Preferably, said antibody has a 5 fold decreased Kd for one isoform of the protein, more preferably the Kd is 10 fold decreased. As a consequence, said antibody is capable of binding to one isoform of the protein whereas it essentially fails to bind to the other isoform.

In another preferred embodiment of the present invention, the amount of co-localized labels is determined by using the method of "scanning for intensely fluorescent targets (SIFT)" (Ref: Bieschke et al. 2000), FRET or high resolution confocal imaging. Preferably, said high resolution confocal imaging is performed with a confocal laser scanning microscope or with a microscope utilizing spinning disc technology.

In another preferred embodiment of the present invention, said monomeric and aggregating proteins are selected from the group consisting of prion protein, Amyloid precursor protein (APP), alpha-synuclein, superoxide dismutase, tau, proteins with a Poly-Q stretch, immunoglobulin, Amyloid-A, transthyretin, Beta2-microglobulin, cystatin C, Apolipoproteine A1, Islet amyloid polypeptide, ANF, gelsolin, insulin, lysozyme, and fibrinogen, and fragments or derivates of said proteins. Preferably, said proteins with a Poly-Q stretch are proteins which have at least 36 consecutive glutamine residues. More preferably, said proteins with Poly-Q stretch are selected from the group consisting of huntingtin and ataxin. Preferably, said fragments or derivatives are selected from the group modified by phosphorylation, glycosylation, proteolytic processing, glycation, oxidation, and nitration. The (poly)peptides mentioned in the present invention may contain one or more carbohydrates, carbohydrate derivatives, lipids, phosphate, sulfate, fatty acids, and nucleotides attached to individual amino acids in the chain. Preferably, said modification is a phosphorylation, glycosylation, proteolytic processing, glycation, oxidation, and nitration. The protein may be a vertebrate or invertebrate protein. Preferably, the protein is a mammalian or avian protein. More preferably, the mammalian protein is selected from primate, human, mouse, rat, bos (cattle), sus (pig), and sheep. In particular cases it may be preferable to use mixed isoforms, i.e. for example the aggregated form PrP^{sc} derived from human and the monomeric form derived from mouse. The proteins may be isolated from an animal or from tissue culture or may be prepared recombinantly. It is envisaged by the inventors, that the proteins may be chemically modified or treated by enzymes such as protease or glycosidases in order to improve handing in the assay system.

In a more preferred embodiment of the present invention, the monomeric protein is prion protein and the aggregated protein is PrP^{sc}.( Prusiner 1998)

In another preferred embodiment of the present invention, said compound is selected from the group consisting of: 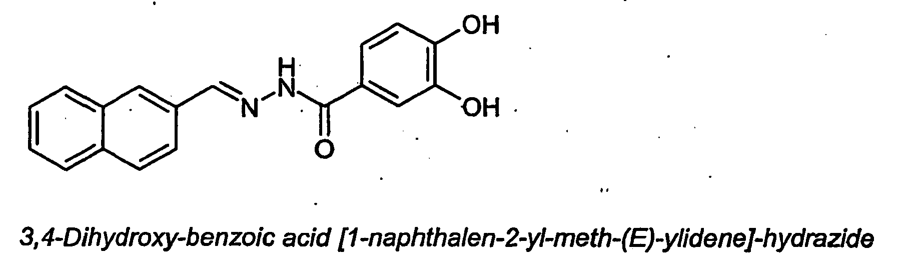 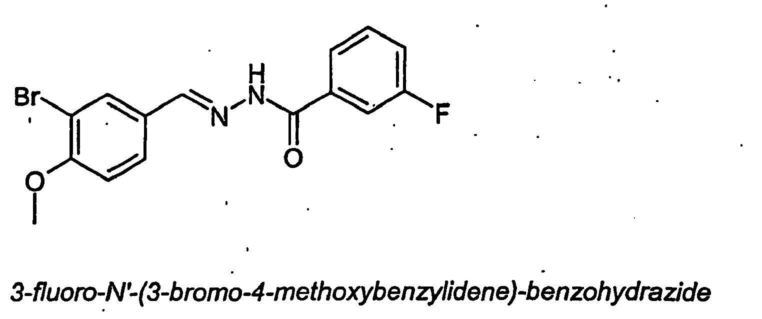 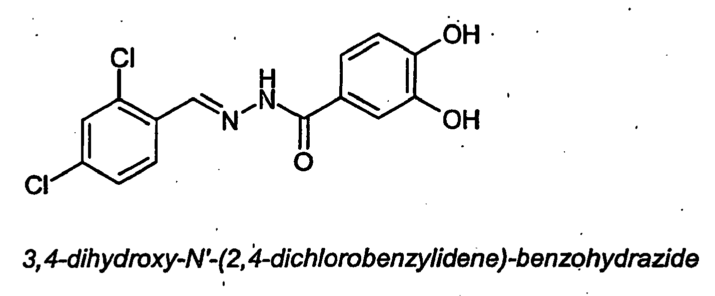 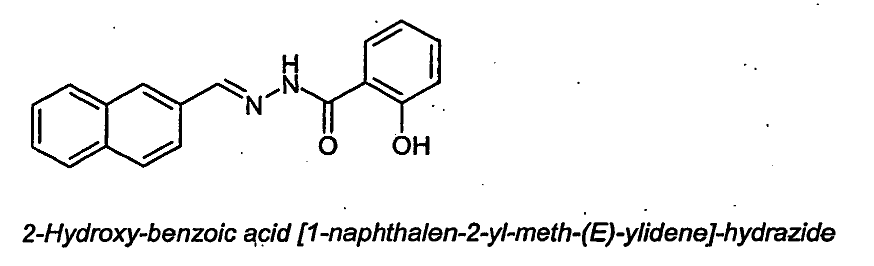 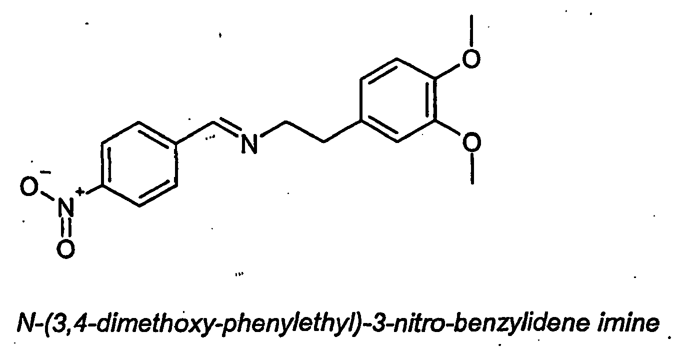 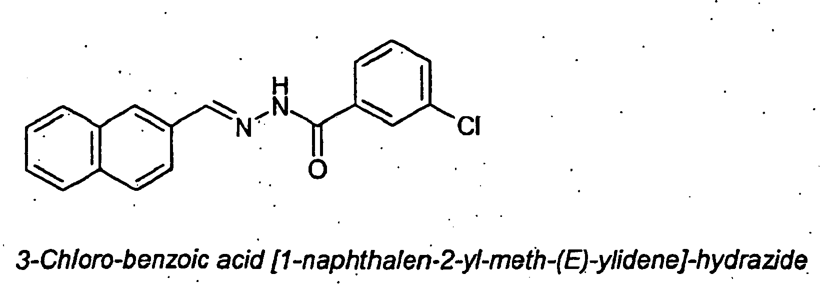 and

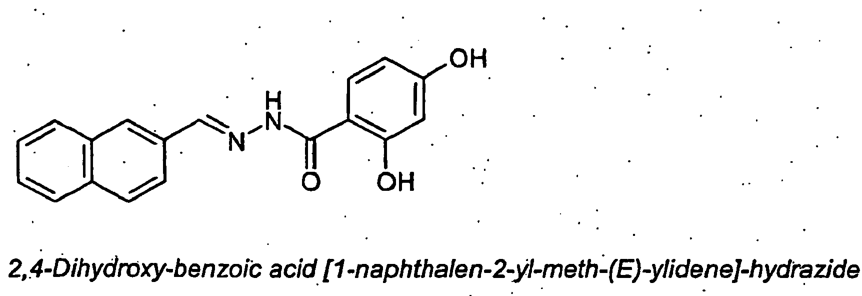

Also described is the compound:

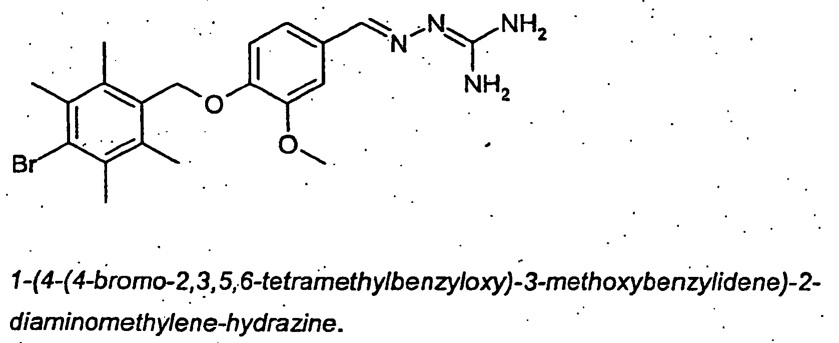

The structure formulas shown above and throughout the specification of the present invention do not show all hydrogen atoms. The skilled person knows that this representation does not mean that the actual compounds contain no hydrogen atoms.

In a more preferred embodiment of the invention, efficacy of said compound is further improved by derivatization. Derivatization refers to the generation of chemically related compounds which have modifications in at least one position of the molecule.

The present invention also relates to a method of selecting compounds with in vivo efficacy in the treatment of diseases linked to protein aggregation and/or neurodegenerative diseases, comprising (a) administering a candidate compound as defined in the present invention to a cell culture or a non-human animal having the aggregatable isoform of the protein as defined in the present invention; (b) quantifying the amount of observable aggregates; and (c) identifying and selecting a compound which is capable of reducing aggregates or the formation of aggregates of said protein.

This method of the present invention allows testing candidate compounds in vivo, i.e. in cells within or outside of a living organism. Testing candidate compounds in vivo is used in example 5 (vide infra). Testing candidate compounds in vivo gives important additional information including data regarding toxicity, stability in the presence of a complex chemical environment, and ability to reach the location where a desired molecular effect is achieved.

Preferably, the compound is administered in various concentrations in order to determine a concentration at which an effect on protein aggregation can be observed and in order to calculate the EC50. EC50 refers to the (molar) concentration of a compound, which produces 50% of the maximum possible response for that compound.

The present invention also relates to the use of a compound for inhibiting protein aggregation in vitro, or ex vivo, wherein said compound is selected from the group represented by formula (I) 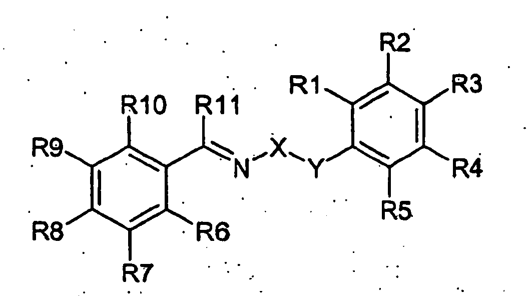 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R12 (if X is CH-R12), R13 (if X is N-R13) and R14 (if Y is CH-R14) are independently selected from hydrogen, alkyl, cycloalkyl heterocycloalkyl, or hydroxyalkyl, each of which is optionally substituted

Also described is the above outlined use in a non-human animal

Also described is the use of a compound for inhibiting protein aggregation in vitro, in a non-human animal or ex vivo, wherein said compound is selected from the group represented by formula (II) 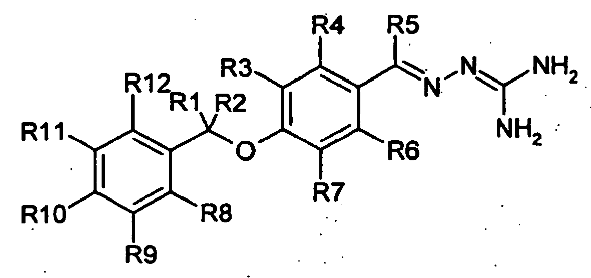 wherein in formula (II):
R1 and R2 are independently selected from hydrogen, alkyl, cycloalkyl heterocycloalkyl, or hydroxyalkyl, each of which is optionally substituted;
R3, R4, R5, R6, R7, R8, R9, R10, R11 and R12 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted.

In a preferred embodiment of the present invention, said compound is detectably labeled. Any label described in the present invention (vide supra) may be used.

In another preferred embodiment of the present invention, two or more of said compounds are used simultaneously.

Moreover, the present invention relates to the use of a compound for the preparation of a pharmaceutical composition for the treatment of a disease linked to protein aggregation and/or neurodegenerative disease, wherein said compound is selected from the group represented by formula (I) 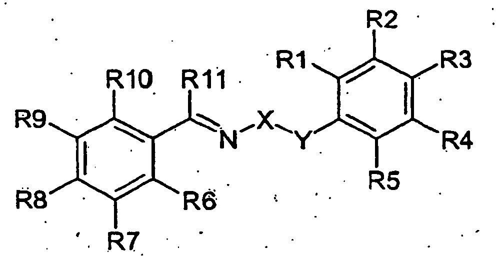 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, vitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R12 (if X is CH-R12), R13 (if X is N-R13) and R14 (if Y is CH-R14) are independently selected from hydrogen, alkyl, cycloalkyl heterocycloalkyl, or hydroxyalkyl, each of which is optionally substituted

Also described is the use of a compound for the preparation of a pharmaceutical composition for the treatment of a disease linked to protein aggregation and/or neurodegenerative disease, wherein said compound is selected from the group represented by formula (II) 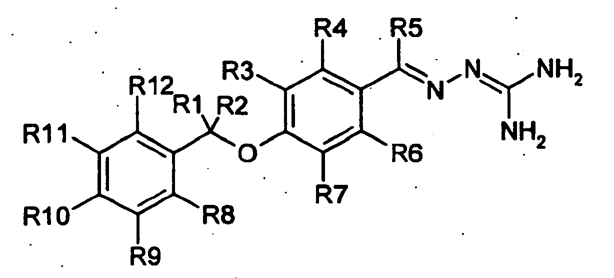 wherein in formula (II):
R1 and R2 are independently selected from hydrogen, alkyl, cycloalkyl heterocycloalkyl, or hydroxyalkyl, each of which is optionally substituted;
R3, R4, R5, R6, R7, R8, R9, R10, R11 and R12 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy; heteroaryloxy, arylalkoxy, heteroarylalkoxy, haloalkoxy, carboxy, carbonylamido and alkylthiol, each of which is optionally substituted.

In a more preferred embodiment of the invention, said disease linked to protein aggregation is characterized by the presence of aggregated forms of at least one protein or a fragment or derivative thereof, wherein this protein is selected from the group consisting of prion protein, Amyloid precursor protein (APP), alpha-synuclein, superoxide dismutase, tau, proteins with a Poly-Q stretch, immunoglobulin, Amyloid-A, transthyretin, Beta2-microglobulin, cystatin C, Apolipoproteine A1, Islet amyloid polypeptide, ANF, gelsolin, insulin, lysozyme, and fibrinogen. Preferably, said proteins with a Poly-Q stretch are proteins which have at least 36 consecutive glutamine residues. More preferably, said proteins with Poly-Q stretch are selected from the group consisting of huntingtin and ataxin. It is known to the skilled person that said proteins may exist in various isoforms including proteins modified by phosphorylation, glycosylation, proteolytic processing and the like. The term "at least one refers to the fact known to the skilled person that diseases can be linked to the presence of more than one protein in the aggregated form. For example, in Alzheimer's disease aggregates of fragments of APP and aggregates of tau are usually detectable.

As used herein, the term "neurodegenerative diseases" comprises diseases such as Alzheimer's disease, prion disease, Parkinson's disease, multiple system atrophy, Diffuse Lewy body disease, frontotemporal dementia, amyotrophic lateral sclerosis, Huntington disease's, spinocerebellar ataxias and other Poly-Q diseases, hereditary cerebral amyloid angiopathy, familial amyloid polyneuropathy. Moreover, as used herein, the term "protein aggregation diseases" refers to diseases manifested predominantly outside the nervous system and includes diseases such as primary systemic amyloidosis (AL amyloidosis), reactive systemic amyloidosis (AA amyloidosis), type II diabetes, injection-localized amyloidosis, beta-2 microglobulin amyloidosis, hereditary non-neuropathic amyloidosis, Finnish hereditary systemic amyloidosis.

In a preferred embodiment of the invention, said disease is selected from the group consisting of Alzheimer's disease, prion disease, Parkinson's disease, multiple system atrophy, Diffuse Lewy body disease, frontotemporal dementia, amyotrophic lateral sclerosis, Huntington disease's, spinocerebellar ataxias and other Poly-Q diseases, hereditary cerebral amyloid angiopathy, familial amyloid polyneuropathy, primary systemic amyloidosis (AL amyloidosis), reactive systemic amyloidosis (AA amyloidosis), type II diabetes, injection-localized amyloidosis, beta-2 microgiobulin amyloidosis, hereditary non-neuropathic amyloidosis, Finnish hereditary systemic amyloidosis.

In a more preferred embodiment of the invention, said prion disease is selected from Creutzfeldt-Jakob disease, variant Creutzfeldt-Jakob disease, genetic human prion disease, Bovine Spongiform Encephalopathy (BSE) and Scrapie.

In a preferred embodiment of the invention said compound is selected from the group consisting of: 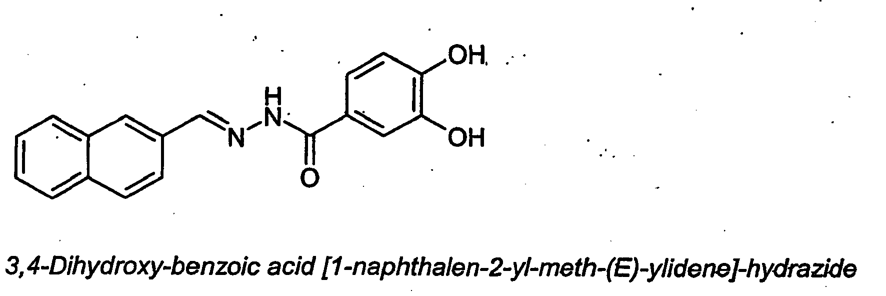 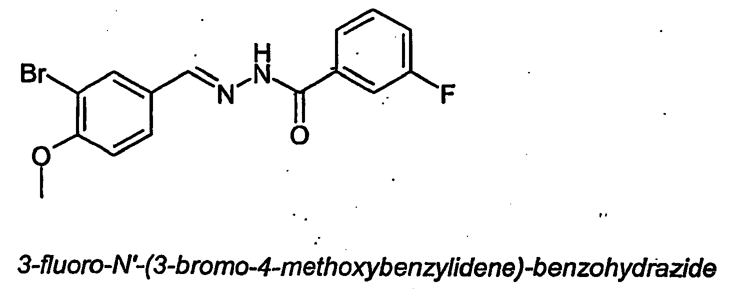 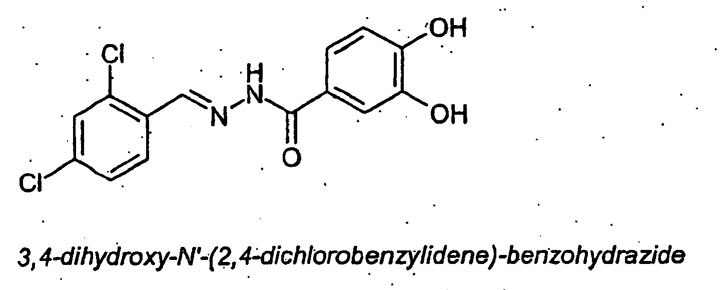 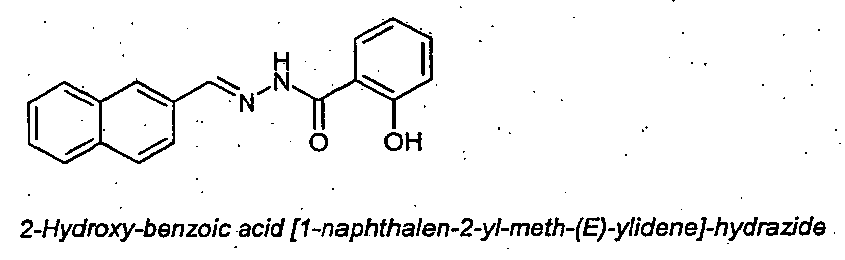 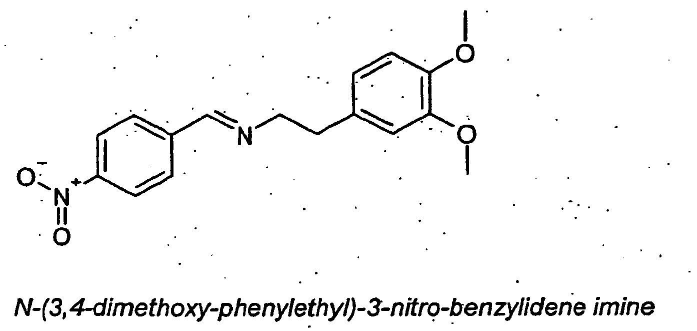 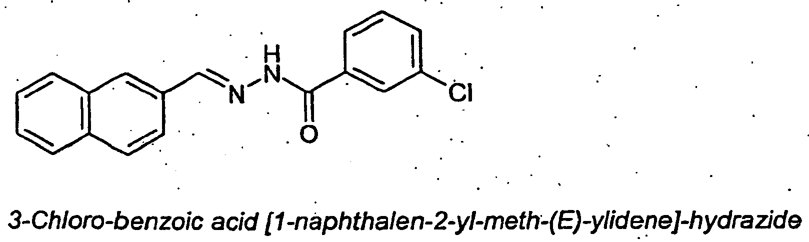 and

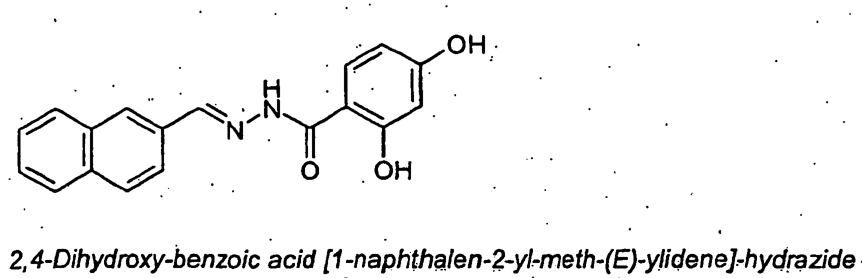

Also described is the compound :

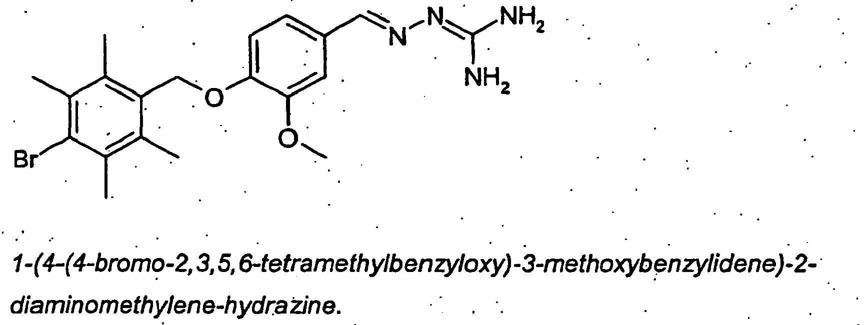

The present invention also relates to a pharmaceutical composition comprising a compound and optionally a pharmaceutically acceptable carrier or excipients, wherein said compound is selected from the group represented by formula (I) 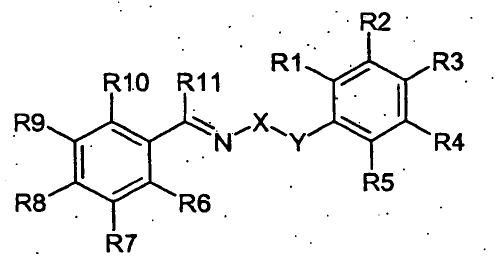 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R13 (if X is N-R13) and R14 (if Y is CH-R14) are independently selected from hydrogen, alkyl, cycloalkyl heterocycloalkyl, or hydroxyalkyl, each of which is optionally substituted

Also described is a pharmaceutical composition comprising a compound and optionally a pharmaceutically acceptable carrier or excipients, wherein said compound is selected from the group represented by formula (II) 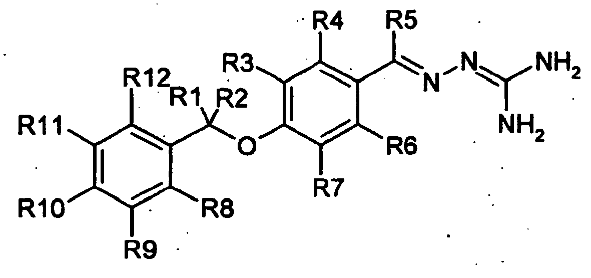 wherein in formula (II):
R1 and R2 are independently selected from hydrogen, alkyl, cycloalkyl heterocycloalkyl, or hydroxyalkyl, each of which is optionally substituted;
R3, R4, R5, R6, R7, R8, R9, R10, R11 and R12 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted.

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.

The skilled person knows that the effective amount of pharmaceutical composition administered to an individual will, inter alia, depend on the nature of the compound. For example, if said compounds is a (poly)peptide or protein the total pharmaceutically effective amount of pharmaceutical composition administered parenterally per dose will be in the range of about 1 µg protein /kg/day to 10 mg protein /kg/day of patient body weight, although, as noted above, this will be subject to therapeutic discretion. More preferably, this dose is at least 0.01 mg protein /kg/day, and most preferably for humans between about 0.01 and 1 mg protein /kg/day for the peptide. If given continuously, the pharmaceutical composition is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. If the compound is of less molecular weight, as is the case with most of the compounds disclosed in the present invention, lower doses may be administered at different intervals. The particular amounts may be determined by conventional tests which are well known to the person skilled in the art.

Pharmaceutical compositions of the invention may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, sernisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained release systems. Suitable examples of sustained-release compositions include semipermeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., Blopolymers 22:547-556 (1983)), poly (2- hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D- (-)-3-hydroxybutyric acid (EP 133,988). Sustained release pharmaceutical composition also include liposomally entrapped compound. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

Moreover, the present invention also relates to a diagnostic composition comprising a compound selected from the group represented by formula (I) 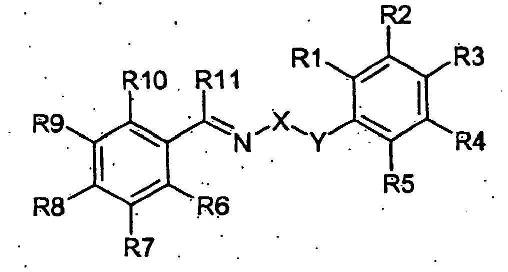 wherein in formula (I):
X is N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R13 (if X is N-R13) and R14 (if Y is CH-R14) are independently selected from hydrogen, alkyl, cycloalkyl heterocycloalkyl, or hydroxyalkyl, each of which is optionally substituted

Also described is a diagnostic composition comprising a compound selected from the group represented by formula (II)
wherein in formula (II):
R1 and R2 are independently selected from hydrogen, alkyl, cycloalkyl heterocycloalkyl, or hydroxyalkyl, each of which is optionally substituted;
R3, R4, R5, R6, R7, R8, R9, R10, R11 and R12 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted.

In a preferred embodiment of the invention, said compound is detectable or detectably labelled. Any of the labels described in the present invention may be used (vide supra). It is understood in accordance with the present invention that a compound is detectable or detectably labelled if its presence can be monitored by conventional techniques such as spectrometry, chromatography, ELISA assay, detection of radioactive emission, preferably by scintillation counting or gamma counting.

In another preferred embodiment of the present invention, said compound is selected from the group consisting of: 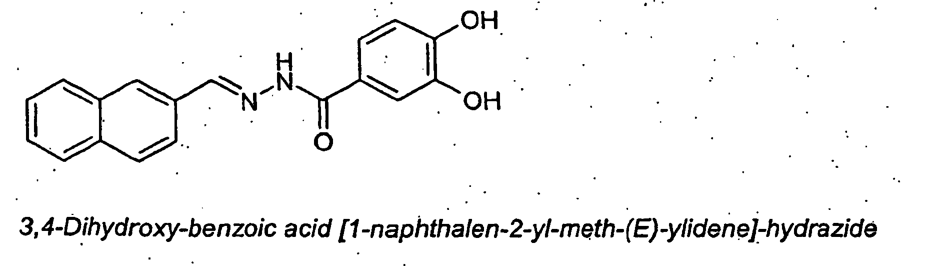 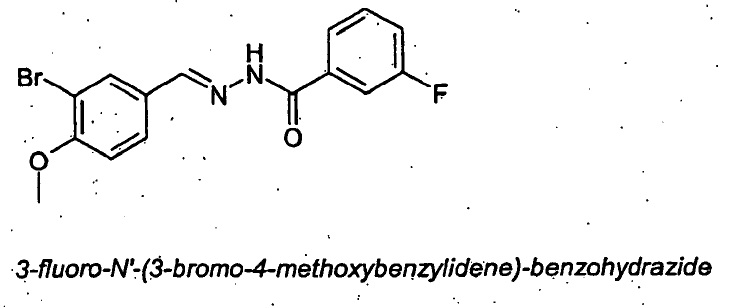 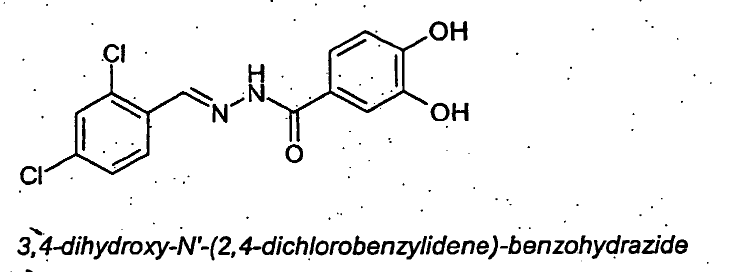 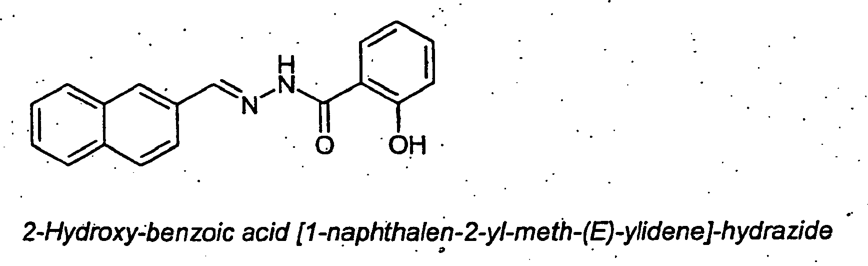 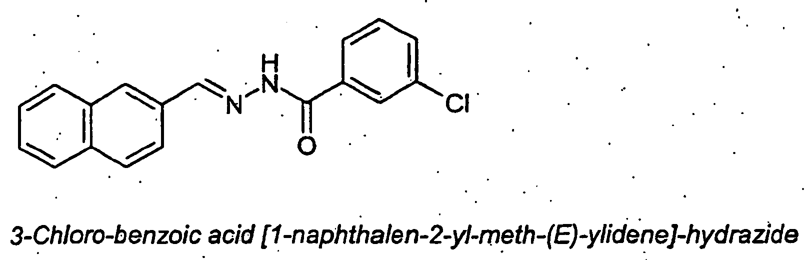 and

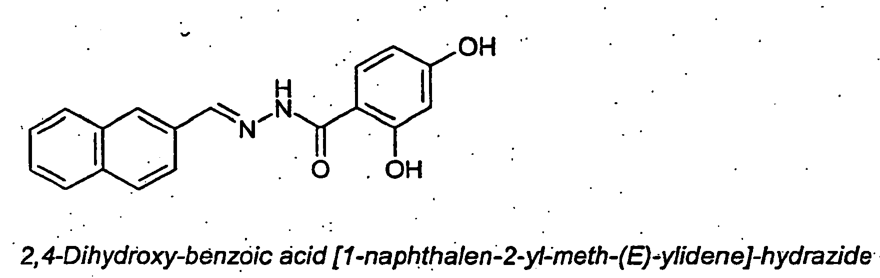

Also described are compounds selected from the group consisting of: 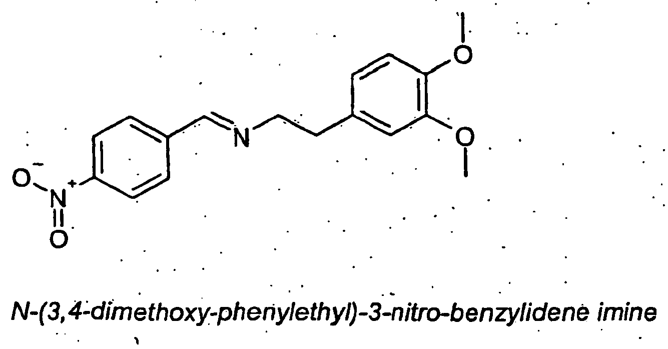 and

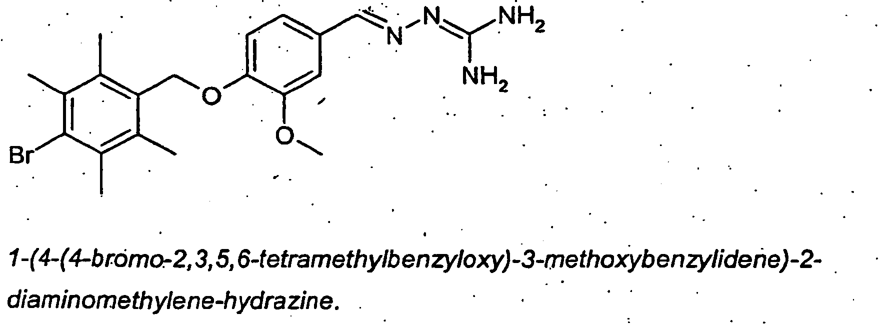

Finally, the present invention relates to a kit comprising the compound as defined in the present invention and, in addition, an antibody or antibody fragment specifically binding to said compound; and/or monomeric or aggregate protein as defined in the present invention; and/or monomeric or aggregated protein as defined in the present invention, optionally complexed with said compound and instructions for use, in one or more container.

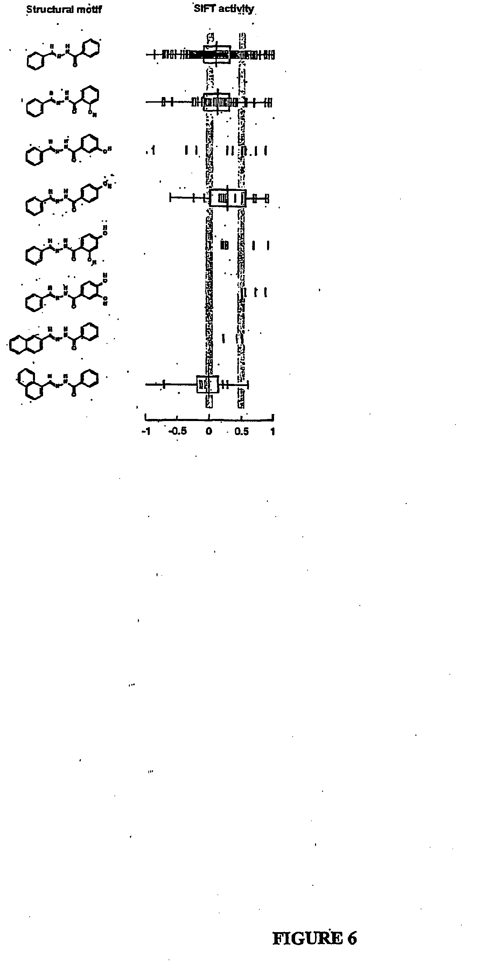

The figures show:

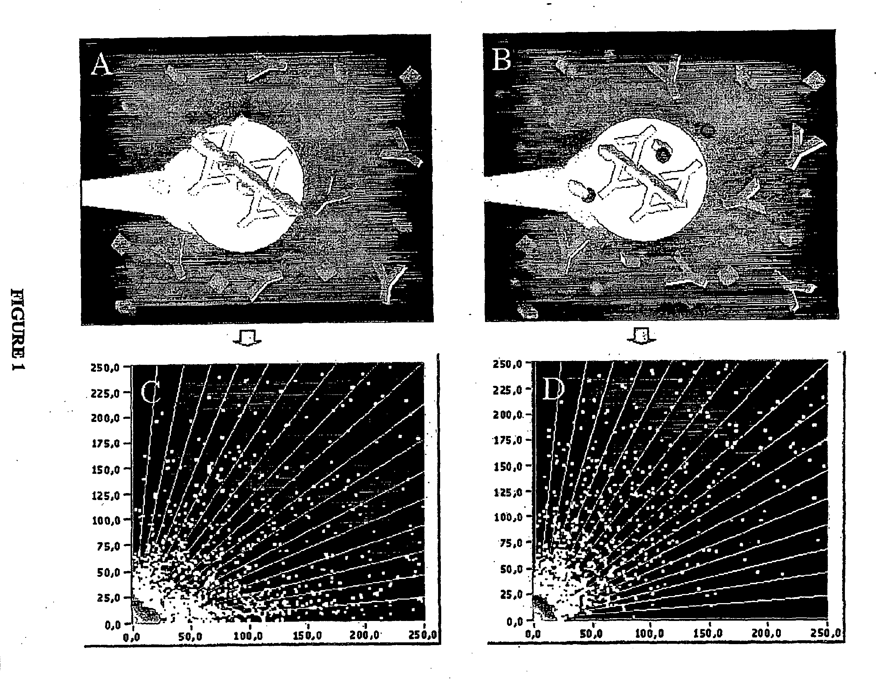

- **Figure 1:**: Schematic representations of the assay components without and with therapeutic compound, respectively. (A and B) Antibodies to human PrP are depicted as red Ys. Mouse rPrP is symbolised by green cubes. Human PrP-Sc aggregates are drawn as chains of pink cubes. In B therapeutic compounds binding to rPrP are symbolized by blue spheres.(C and D) Two-dimensional fluorescence intensity distribution histograms of in vitro fluorescence correlation assays without and with 17 µM DOSPA, respectively. Red fluorescence intensity of single 40 µs events (bins) is given on the vertical axis as number of photons, the green fluorescence intensity of the events is indicated on the horizontal axis. The diagram is divided into eighteen sectors of events with comparable relation between red and green intensity. Events with low intensities (e.g. less than 20 red or green photons per 40 µs) are excluded in a nineteenth sector around the origin of the diagram. The number of events with identical red and green intensities is colour-coded ranging from yellow through blue and green up to white for increasing numbers of events. The presence of the inhibitory compound DOSPA shifts the appearance of high intensity events away from the "green" sectors at the bottom to the "red" sectors at the left.

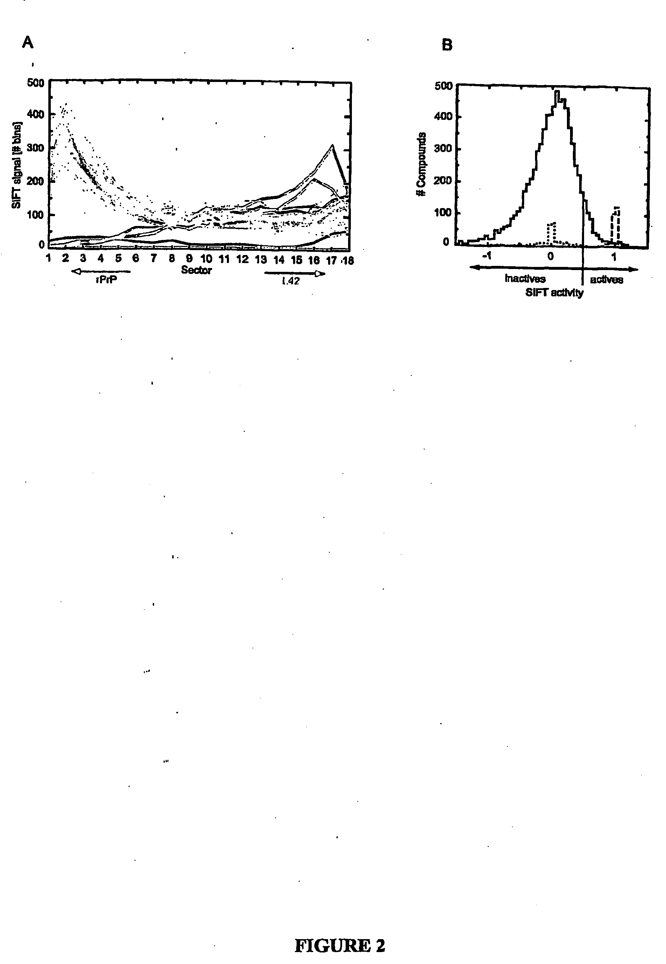

- **Figure 2:**: Primary SIFT screening: (A) Fluorescence intensity distribution of one primary screening experiment for 80 compounds and 8 control reactions. The number of high-intensity bins found in each of the eighteen sectors is plotted for each compound and control reaction. Controls containing no PrPSc - aggregates and compounds (only rPrP and mAb mix) are blue grey. Controls containing 17 µM DOSPA are red.(B) SIFT primary activity values for the DIVERSet1 library. Library compounds (solid), negative controls (dotted) and positive DOSPA controls (dashed). The activities of negative and positive controls are narrowly distributed around 0 and 1 respectively and are clearly separated. A selectable boundary value (here 0.5) splits the DIVERSet1 compounds into inactives and actives.

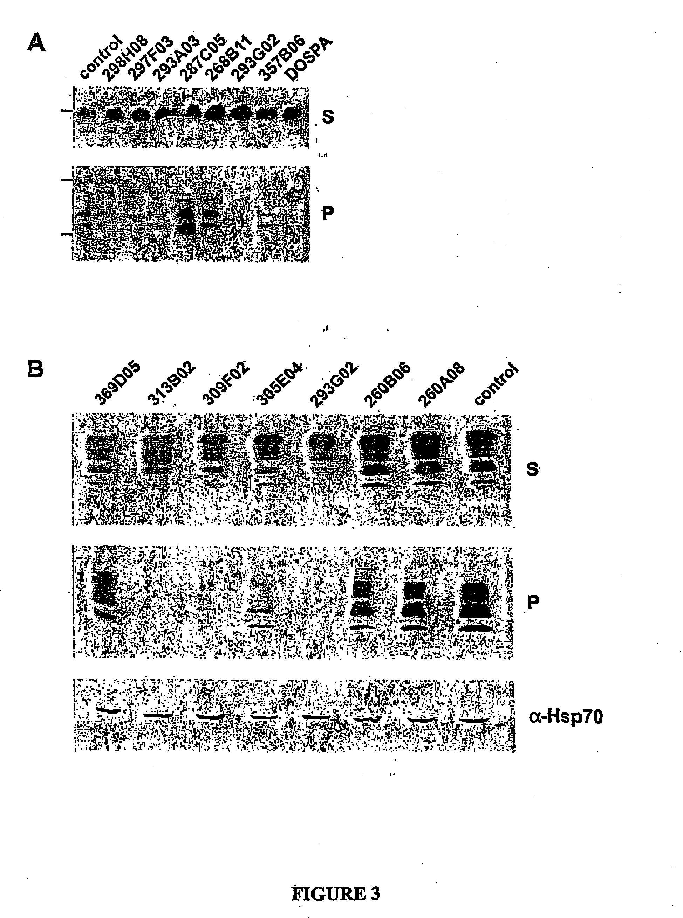

- **Figure 3:**: Inhibition of PrPSc formation in scrapie-infected N2a cells. (A, B) ScN2a cells were cultivated for three days and then compounds were added at a concentration of 15 (A) or 10 mM (B). After cultivation for additional two days cells were lysed in detergent buffer, and PrP present in the detergent-soluble (S) and -insoluble (P) was analyzed by Western blotting using the anti-PrP antiserum A7. The relative amount of the cytosolic chaperone Hsp70 present in the detergent soluble fraction was analyzed in parallel (B, µ-HSP70). Incubation with DOSPA served as a positive control for anti-prion activity.

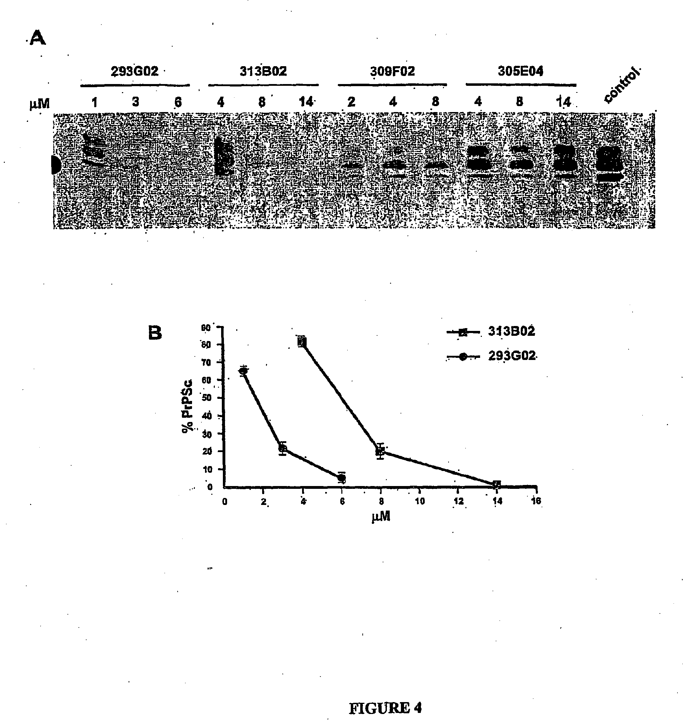

- **Figure 4:**: Dose response analysis of new anti-prion drugs. (A) Selected compounds were tested at different concentrations as described under Fig. 4. The detergent-insoluble fraction was treated with proteinase K prior to Western blot analysis. (B) Quantitative analysis of the experiments shown under A. To quantify the reduction of PrPSc by the different compounds, the relative amount of PrPSc present in control cells was set as 100%. Reduction was calculated from three independent experiments.

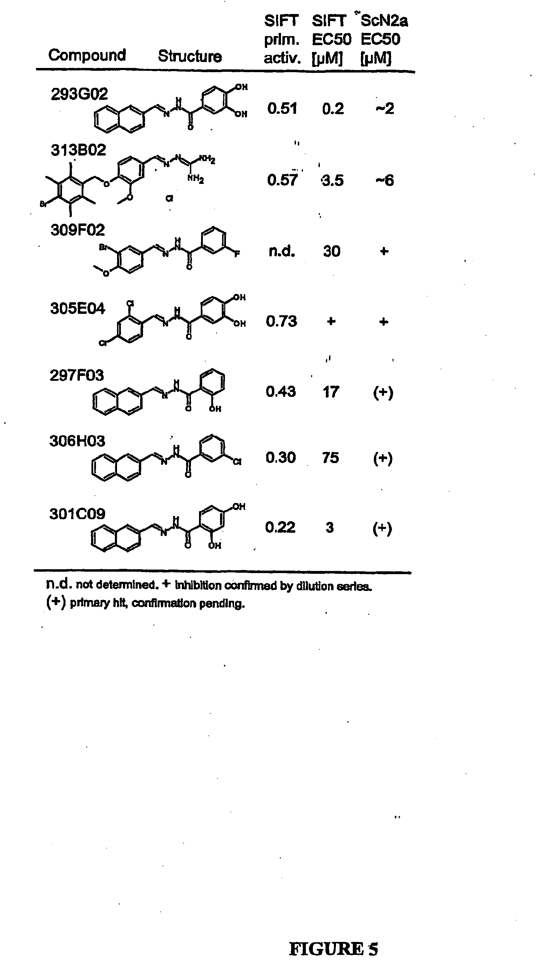

- **Figure 5:**: Compounds with cell culture activity. The molecular structures of these substances and their activities in the three steps of the combined screening approach are shown. The first of the three columns shows the activities in the primary SIFT screening, where they were all selected as primary SIFT hits. All these substances were validated in SIFT dilution series, and, where possible, EC50 values were determined. The last column combines the results of several steps in the cell culture system. Evidently, six of these compounds share a N'-benzylidenebenzohydrazide core.
Compound names are: 293G02: 3,4-dihydroxy-N'-(naphthalen-2-ylmethylene)-benzohydrazide, 313B02: 1-(4-(4-bromo-2,3,5,6-tetramethylbenzyloxy)-3-methoxybenzylidene)-2-diaminomethylenehydrazine, 309F02: 3-fluoro-N'-(3-bromo-4-methoxybenzylidene)-benzohydrazide, 305E04: 3,4-dihydroxy-N'-(2,4-dichlorobenzylidene)-benzohydrazide, 297F03: 2-hydroxy-N'-(naphthalen-2-ylmethylene)-benzohydrazide, 306H03: 3-chloro-N'-(naphthalen-2-ylmethylene)-benzohydrazide, 301 C09: 2,4-dihydroxy-N'-(naphthalen-2-ylmethylene)-benzohydrazide
- **Figure 6:**: Structure-activity relationships for N'-benzylidene-benzohydrazide derivatives. SIFT primary activities of eight substance classes are shown. The substance classes are characterized in that they contain the depicted structure motifs. The boxes in-between the activity distributions mark median and quartiles.

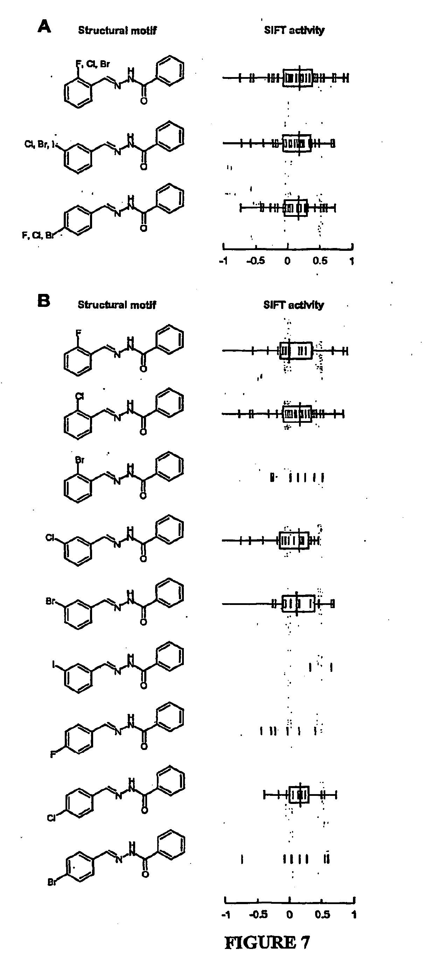

- **Figure 7:**: Structure-activity relationships for halogen-substituted N'-benzylidenebenzohydrazide derivatives. In **A** SIFT primary activities of three substance classes are shown, that are characterized by the substitution of halogens at the benzyl ring of the benzylidene moiety at the N'-position of the NBB core. In **B** these classes are split up according to the type of the halogen atom used for substitution. The boxes and large vertical bars within the strongly occupied activity distributions mark the median and the quartiles; that statistics has been omitted for classes containing only a few members. -

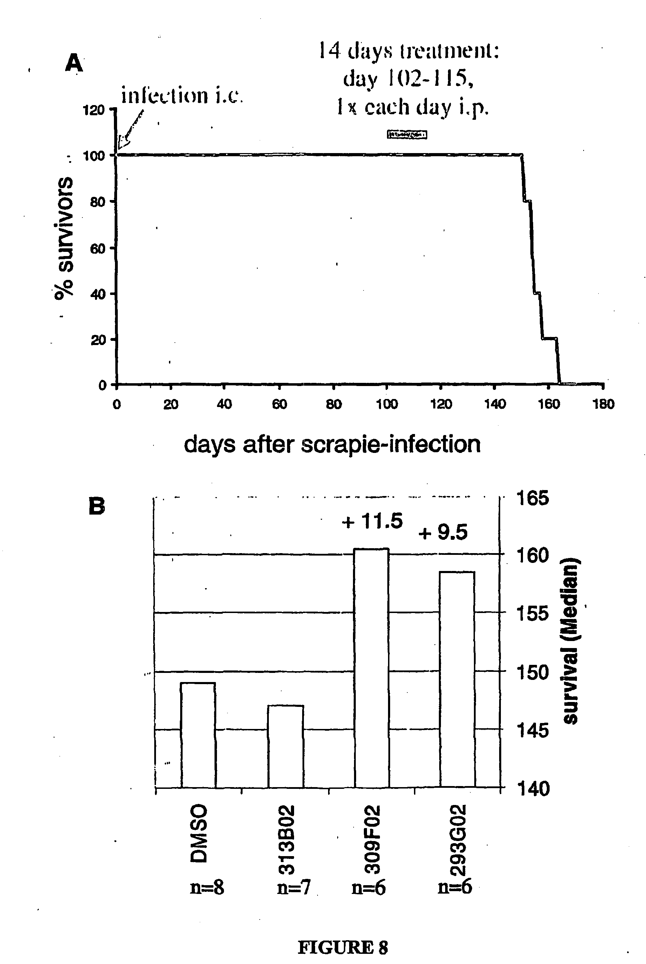

- **Figure 8:**: A. Therapeutic scheme of drug application during the course of the disease measured as % of surviving animals at a given day after intracerebral infection with RML-scrapie. B. Median of the survival times of the four experimental groups of animals, which were either treated with one of the three compounds or with the vehicle (75% DMSO) only

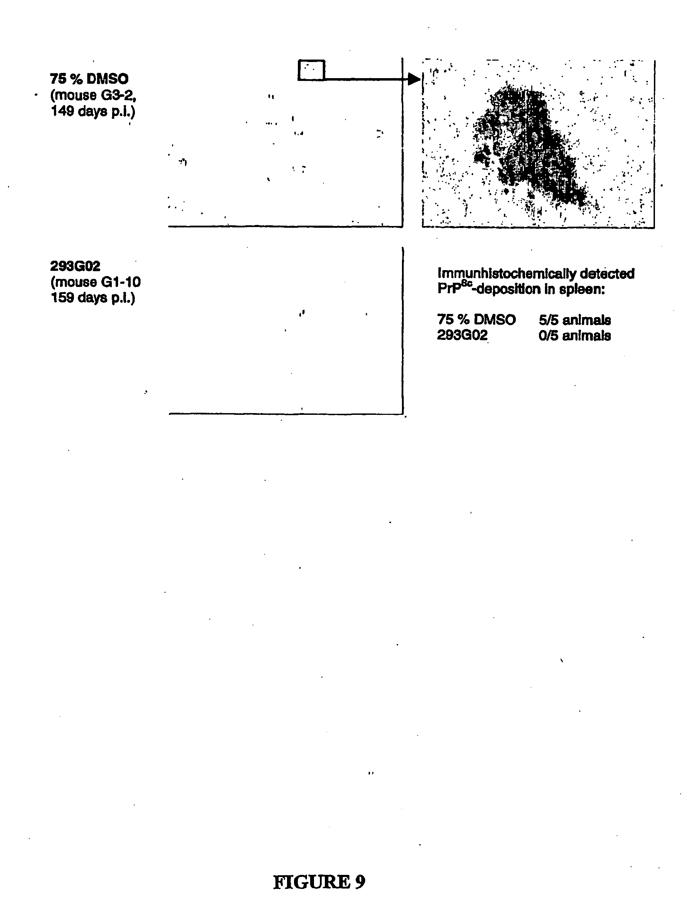

- **Figure 9:**: Upper panel: Example of a tissue slice of the spleen of a mock-treated animal immunohistochemically stained for PrP-Sc deposits at lower (left) and higher (right) magnification. PrP-Sc deposits are detectable by the red colour of the PK-resistant amyloid. Lower panel: Example of a tissue slice of the spleen of a animal treated with substance 293G02 and immunohistochemically stained for PrP-Sc deposits. Here no PrP-Sc deposits can be detected in the low magnification overview of the origan.

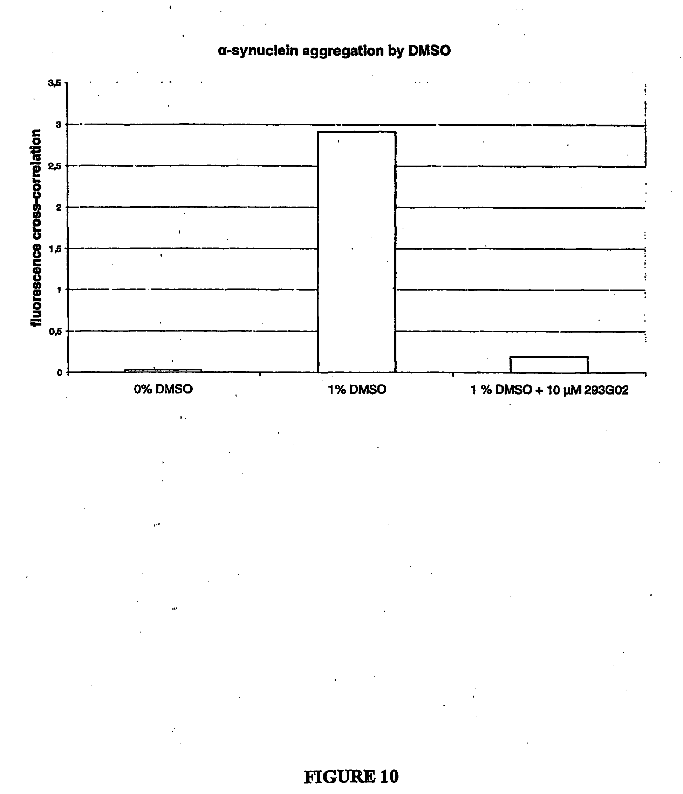

- **Figure 10:**: Median of the fluorescence cross-correlation measured for three independent samples of a mixture of two differently labeled α-synuclein monomers receiving the same treatment. Three samples each were treated with or without DMSO or DMSO plus compound 293G02, respectively. Each sample was measured seven times during the period 3-5 hours after start of the treatment. Increasing cross-correlation indicates formation of more and bigger aggregates.

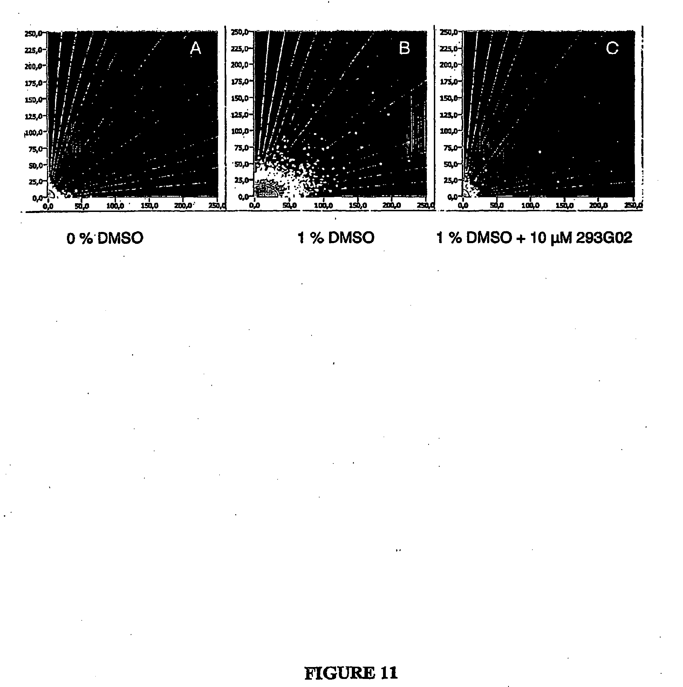

- **Figure 11:**: Examples of two-dimensional fluorescence intensity distribution histograms for samples treated either without (**A**) or with DMSO (**B**) or with DMSO plus compound 293G02 (**C**). Red fluorescence intensity is indicated on the vertical axis, green fluorescence intensity on the horizontal axis. The intensity is given in photons counted per 40 µs time interval (bin). The number of bins measured having a particular fluorescence intensity distribution is color coded with yellow for single events through blue, green, and white for increasing number of bins with the same intensities.

The examples illustrate the invention:

### Example 1: The SIFT anti-prion assay

To test the inhibitory effect of drugs on the association between PrPC and PrPSc in a high-throughput and high-content screening assay, we applied the "Scanning for Intensely Fluorescent Targets" (SIFT-) technique (Bieschke et al. 2000), which utilises an inverted dual color confocal microscope setup with single photon detectors for two colors of fluorescent light. Samples are prepared in 96- or 384-well microtiter plates with cover slide glass bottoms. The assay mixture consists of recombinant mouse PrP (rPrP), the monoclonal antibody (mAb) L42, which does not recognize mouse PrP but human PrP, and PrP-Sc aggregates prepared from human CJD brain. The rPrP and mAb molecules are labeled with green and red fluorophores, respectively. Binding of several rPrP and mAb molecules to the PrPSc aggregates will result in the formation of ternary complexes exhibiting many red and green attached fluorophores (Fig. 1). Such aggregates can be identified and analyzed by the SIFT method, because they simultaneously cause high intensities in both fluorescence channels. The distribution of the red and green fluorescence intensities can be assessed by a two-dimensional fluorescence intensity histogram. Whenever an inhibitor of the association between rPrP and PrPSc is included in the assay, the green fluorescence intensity of the ternary aggregates should decrease (Fig. 1). The color distribution of the aggregates in the 2D-histogram will then be shifted towards the "red" sectors of that histogram, as shown in Fig. 1D for a sample containing 17 µM DOSPA, a cationic lipid that has previously been found to inhibit PrPSc formation in scrapie-infected mouse cells in cell culture.

### Example 2: Primary SIFT-screening of 10 000 compounds

The application of this assay system to a library of 10 000 diverse, drug-like compounds (ChemBridge DIVERSet1) in 96-well microtiter plates is exemplified in Fig. 2A showing the results of a primary screening of one microtiter plate containing 80 compounds from the library and 8 control samples. Three of these were negative controls without any additional compounds and three positive controls containing 17 µM DOSPA as well as two controls without CJD-rods and compounds (serving to check the absence of aggregation in the antibody and rPrP mixture).

As can be seen in this figure, the three samples containing DOSPA showed reduced SIFT signal in those sectors which monitor signals of aggregates predominantly labelled with green rPrP. This indicates that in these controls less rPrP had bound to CJD prion rods. Because the prion rods are marked by the red antibody labels, their fluorescence still generates SIFT signal in the "red" sectors. Most of the compounds did not influence the distribution of the SIFT signal. But some of the DIVERSet compounds lowered the number of aggregates detected in the "green" sectors. The SIFT curves of these samples are shifted towards the DOSPA controls. Thus, the corresponding compounds can be considered as primary hits for potential anti-prion drugs. Occasionally some technical problems generated artefacts, which obscured an entire measurement of a whole mircotiter plate. Apart form this, only about 7 % of the undisturbed measurements had to be treated as outliers and were unsuitable for the automated SIFT analysis, mostly because of intrinsic fluorescence of the tested compounds. This rather low percentage of compounds untreatable by the SIFT assay was unexpected and underscores the versatility and robustness of the assay. The identification of problematic measurements and compounds is facilitated by the high-content nature of the SIFT assay data. For each sample several fluorescence parameters are recorded simultaneously, like for instance the mean fluorescence intensities for each color channel. These are displayed together with the sums of the high-intensity events in each sector of the color distribution histogram. Therefore samples with high intrinsic fluorescence can easily be sorted out.

### Example 3: SIFT primary hits

For a compound to be classified as a primary hit, we analyzed the sum of bins in the "green" sectors 1-5 and defined a cut-off value of approximately 50 % of the effect of DOSPA compared to the untreated controls as the minimum effect necessary. With this definition we obtained 256 primary hits from the library after a single round of screening with our SIFT assay.

For the automated quantification of the SIFT-screening data, we have subsequently developed a software module, which additionally assigns primary SIFT activity values to the tested compounds (see the Methods section for details). Fig. 2B shows the distribution of these activity values over the whole DIVERSet1 library.

### Example 4: Validation of primary hits by dilution series

The primary hits were checked for dose-dependent inhibition of PrPC - PrPSc association using duplicate six point dilution series (0.1-100 mM) of each compound in the SIFT assay. For 80 compounds these dose-response curves confirmed their concentration dependent inhibitory activity. Compared to the effect of 17 µM DOSPA, half-maximal inhibition of binding of rPrP to prion rods was observed at EC50 values in the range of 0.3 to 60 µM.

### Example 5: Validation of hits in a cell culture model of prion diseases

After the identification of promising compounds in our high-throughput in vitro assay, we evaluated their anti-prion activity in a biological system. Besides infections of laboratory animals, there are cell culture models of prion propagation. So far, only a limited number of cell lines could be efficiently infected with scrapie prions, among them N2a cells. The infected cell lines (e.g. ScN2a) are characterized by the formation of detergent insoluble and PK-resistant PrPSc and the propagation of infectious prions (Borchelt et al. 1992, Butler et al. 1988, Caughey & Raymond 1991, Schätzlet al. 1997). These cell culture models proved useful to study the biogenesis of PrPC and its conversion into PrPSc. Moreover, we and others have successfully used this system to identify compounds which interfere with PrPSc propagation (Gilch, S. et al. 2001, Kiachopoulos et al. 2004, Tatzelt et al. 1996, Winklhofer et al. 2001). Thus, we used scrapie-infected N2a (ScN2a) cells, a model system employed previously for the identification and characterization of anti-prion drugs(Gilch, S. et al. 2001, Kiachopoulos et al. 2004, Tatzelt et al. 1996, Winklhofer et al. 2001).

Three days after plating, the compounds were added to the cell culture medium (15 mM) and the ScN2a cells were cultivated for additional 2 days. After cell lysis in detergent buffer, the samples were fractionated by centrifugation and PrP present in the detergent-soluble (S) and detergent-insoluble fractions (P) was detected by Western blotting. Mock-treated ScN2a cells are characterized by the presence of detergent-soluble PrPC and the accumulation of detergent-insoluble PrPSc, which is present in the pellet fraction (Fig. 3A, control). After incubation with DOSPA, PrPSc is cleared from the infected cells (Fig. 3A, DOSPA). Note that PrPC present in the detergent-soluble fraction is unaffected by DOSPA. In the first screening, exemplified by the Western blot analysis shown in Fig. 3A, we tested all 80 compounds validated by the SIFT dilution series. In the ScN2a cell culture model, 8 compounds interfered with the accumulation of PrPSc without showing any overt signs of cytotoxicity.

Six of the compounds active in the first round of cell culture analysis were available for further evaluation. These compounds were re-tested at a concentration of 10 mM. Notably, the relative amount of detergent-soluble PrPC and of cytosolic Hsp70 was not affected, indicating that the compounds do not affect protein synthesis in general (Fig. 3B). Four compounds showed reproducible significant depletion of PrPSc and were thus selected for a further dose response analysis (Fig. 4A). For this analysis the detergent-insoluble pellet fraction was incubated with proteinase K (PK) prior to the Western blot analysis to specifically monitor the disappearance of detergent-insoluble and PK-resistant PrPSc. It turned out that the compounds 293G02 and 313B02 induced clearance of PrPSc from the cells with an EC50 of about 2 and 6 mM, respectively (Fig. 4B).

### Example 6: New anti-prion compounds

Fig. 5 shows the structures of compounds with activity against PrPSc propagation in the cell culture analysis. Six of these compounds (293G02, 309F02, 305E04, 297F03, 306H03, and 301C09) share a common N'-benzylidene-benzohydrazide (NBB) core structure (cf. first row of Fig. 6), whereas only 471 of the 10000 compounds in the DIVERSet1 library contain this motif, suggesting a relevant structure-activity relationship.

Therefore, we investigated the influence of different substitutions around the NBB core structure on the activity found in the primary screening with the SIFT assay. To this aim, we defined a series of motifs with specific substitutions of the NBB core. Each of these structural motifs defines a class of substances from the DIVERSet1 library containing the respective motif. Fig. 6 shows the distributions of the SIFT activities for eight such motifs occurring at different frequencies within the compound library.

The NBB class (Fig.6, first row) contains many inactive compounds, but still its activity distribution is shifted slightly towards anti-prion activity. Regarding substitutions of the benzyl ring of the benzohydrazide core, we analyzed the influence of hydroxy groups. The addition of a hydroxy group at the ortho position (cf. Fig.6, second row) leads to a smaller substance class with a similar mean activity. The classes of compounds with hydroxy groups in meta or in para position (rows three and four) show increased proportions of actives. In particular, all four compounds with a combination of hydroxy groups in meta and para position (row six) exhibit activity in the SIFT assay above the threshold defined for primary hits. Moreover, two of these compounds were among those confirmed active in cell culture. Regarding the N' position of the NBB core, we found a striking effect of naphthalenylmethylene substitutions. All compounds containing this moiety as the naphthalen-2-ylmethylene isomer displayed activity in the SIFT assay, whereas the median activity of compounds containing the naphthalen-1-ylmethylene isomer was close to zero. A significant structure-activity relationship is underscored by the fact that two compounds containing the naphthalen-2-ylmethylene group are found among the cell culture actives. Interestingly, the compound with the strongest cell culture activity (293G02) is characterized by the combination of both motifs associated with high activity in the SIFT assay. The evident correlation between SIFT activity and cell culture activity of these structural motifs indicates the feasibility of using data obtained during primary high-thoughput screening in the SIFT assay for evaluation of structure-activity relationships.

### Example 7: Materials and methods used

Compound library. The library screened contains 10000 compounds and will be called DIVERSet1, because it covers only a part of the larger DIVERSet library (ChemBridge Corp., San Diego, CA). DIVERSet is a collection of rationally selected, diverse, drug-like small molecules. The compounds were supplied in dimethyl sulfoxide (DMSO) solution and on 96-well microtiter plates. A database containing molecular structures and some physico-chemical data for each of the compounds is available at www.chembridge.com.

**Production of recombinant mouse PrP 23-231.** Recombinant PrP 23-231 was produced and purified essentially as described by Liemann et al. (1998), except that for bacterial expression BL21 DE3 RIL E. coli cells (Novagen) were transformed with plasmid pET17b-MmPrP23-231WT31 for mouse PrP23-231. Also, the bacteria were grown to an optical density of 0.5 before protein production was induced by addition of 1mM IPTG and cells harvested two hours later. Then bacteria were lysed by addition of 0.5% Triton X-100 to the lysis buffer and incubation for 30 min at 37°C instead of using a French press. Furthermore, the gel filtration step was replaced by a nickel chelate affinity chromatography step. The final cation exchange chromatography step after refolding was also omitted.

In particular, the PrP prepurified by ion exchange chromatography was subjected to oxidation as described and oxidation was terminated by addition of 0.1 mM EDTA and adjustment of pH to about 6. After addition of 0.1 mM NiCl2 up to 50 mg of PrP were applied to 2 ml chelating sepharose (Pharmacia) precharged with NiCl2 according to the manufacturers recommendations and preequilibrated with buffer A (8 M Urea, 10 mM MOPS pH 7.0). Binding of PrP to Ni-chelate-matrix was performed for at least 3h at room temperature by continuously inverting the mixture. The matrix was transferred to a Polyprep-column (BioRad) and drained from the flow through. The column was washed twice with 5 ml buffer B (8 M Urea, 10 mM MOPS pH 7.0, 500 mM NaCl) and then eluted sequentially with 6 times 5 ml puffer D (7.2 M Urea, 10 mM MOPS pH 7.0, 150 mM NaCl, 50 mM imidazole). Fractions containing purified PrP were pooled, concentrated with a centriprep device and finally diluted 1:50 for refolding into 10 mM MES pH 6.0.

**Fluorescence labelling of antibodies and recombinant PrP. L42** monoclonal antibody (Vorberg et al. 1999)(r-biopharm, Darmstadt, Germany) against human PrP was labeled with Alexa^{™} 647 dye (Molecular Probes, Eugene) according to the manufacturers recomendations. Recombinant mouse PrP 23-231 was labeled with the Alexa^{™} 488 dye (Molecular Probes, Eugene) at a concentration of 17,5 µM in buffer A (20 mM pottasium phosphate buffer at pH 6.0, 0.1 % Nonidet P40) containing 150-300 µM of activated dye (Alexa488 carboxylic acid, succinimidyl ester). The duration of the coupling reaction at room temperature in the dark did not exceed two hours before labelled protein and unreacted dye were separated by gelfiltration through Sephadex G-50 Spin columns or PD10 columns (Amersham BioScience) using buffer A for elution.

**SIFT assay for PrPC - PrPSc association.** PrPSc was prepared from brain of CJD patients according to Safar et al. (1998) and aliquots of the final pellet resuspended in 1xPBS+0,1% sarcosyl solution were diluted fivefold into buffer A (20 mM pottasium phosphate buffer at pH 6.0, 0.1 % Nonidet P40) and sonicated in aliquots of up to 100 µl in a water bath sonicator for 60 s. After centrifugation at 1000 rpm for 1 min the supernatant was diluted 100-fold in buffer A for the assay.

For the assay, compounds (approx. 10 mM in DMSO) from the chemical library were first diluted 10-fold into DMSO. This dilution was again diluted 10-fold into buffer A.

A mixture of labeled mouse rPrP and labeled L42 monoclonal antibody was prepared in buffer A so that the labeled molecules were approximately equally abundant at a concentration of 2 to 6 nM.

In a 20 µl assay volume 8 µl of the rPrP / antibody mixture were mixed with 2 µl of the final dilution of one compound from the chemical library, before 10 µl of the diluted PrP-Sc preparation were added. The samples were loaded onto 96 well plates with cover-glas bottom (Evotec-Technologies, Hamburg, Germany) and measured in an Insight II reader (Evotec-Technologies, Hamburg, Germany) set up for FIDA measurements for five times 15 seconds at excitation energies of 200 µW for the 488 nm laser and 300 µW for the 633 nm laser. Fluorescent light from the two fluorophores in the sample was recorded separately with single photon detectors and incident photons were summed up in 40 µs bins.

The fluorescence correlation data was evaluated using a 2D-SIFT software module (Evotec-Technologies, Hamburg, Germany) for the analysis of two-dimensional fluorescence intensity histogramms by summing up of the numbers of high-intensity bins in each of 18 equally sized sectors. Lower cut-off values for bin intensities for each measurement series of 80 compounds plus 8 controls were manually adjusted from the distributions of control reactions with lower cut-offs normally varying between 15 and 30 photons for red and between 20 and 40 photons for green fluorescence.

Automated SIFT screening analysis and primary activity values. To enable an automated evaluation of the SIFT screening results on the DIVERSet1 library we have developed a software module, that rejects faulty measurements and outliers (e.g. due to compounds interfering with total fluorescence) and assigns activity values to the remaining compounds. The outlier detection rejected around 7% of the compound measurements on valid assay plates.

As explained in the Results section, the assignment of activity values utilizes sums of the events in the green sectors (1-5) of the SIFT histograms. We denote this sum for a test substance by s, and the medians of the control measurements measured for a given microtiter plate by cp (negative control) and dp (positive DOSPA control), respectively. The activity value of a substance is then defined by a(s) = (cp - s)/(cp - dp). It relates the distance cp - s of s from the negative control cp to the distance cp - dp between the negative control and DOSPA. The resulting activity values are located near 0 for ineffective compounds and near 1 for inhibitors of PrPC - PrPSc association (see Fig. 2B). Due to the normalization of the distances cp - s, measurements originating from different plates become comparable, because intensity variations among different plates caused by fluctuations of composition cancel.

**Substructure searches and visualization.** Substructure searches in the DIVERSet data base were performed with the Molecular Substructure Miner (MoSS)20, available at fuzzy.cs.uni-magdeburg.de. The 2D-structures of compounds were visualized using the CACTVS system21, which is available at www2.ccc.unierlangen.de/software/cactvs. For both structural searches and visualization the commercial software package Catalyst 4.7 (Accelrys Inc., San Diego, CA) was used.

**Cell lines and drug treatment. ScN2a** cells were cultivated as described (Winklhofer et al., 2001 b). These cells were established by infecting N2a cells (ATCC No. Ccl 131, Monassas, VA, USA) with an enriched preparation of prions isolated from the brains of mice infected with RML prions. Drugs were dissolved in DMSO (1 mM) and added to the cell culture medium three days after plating.

**Detergent solubility assay and proteolysis** experiments. As described earlier (Tatzelt et al., 1996), cells cultivated for 5 days were washed twice with cold phosphate-buffered saline (PBS), scraped off the plate, pelleted by centrifugation and lysed in cold buffer A (0.5% Triton X-100 and 0.5% sodium deoxycholate [DOC] in PBS). The lysate was centrifuged at 15,000 g for 20 min at 4°C; after boiling in Laemmli sample buffer supernatants and pellets were examined by immunoblotting. For the proteinase K (PK) digestion PK was added to the lysate (1 mg per 50 mg protein) and the samples were incubated at 4°C for 60 min. The reaction was terminated by the addition of Pefabloc SC (Roche, Mannheim, Germany).

**Western blot analysis.** Detergent-fractionated cell lysates were size fractionated by SDS-PAGE and proteins transferred to nitrocellulose (Protran BA 85, Schleicher & Schüll, Dassel, Germany) by electroblotting. PrP was detected as described previously (Winklhofer and Tatzelt, 2000). Polyclonal anti-PrP antiserum A7 was raised against recombinant full-length PrP expressed and purified from bacteria (Winklhofer et al., 2003). The antibody against Hsp70 was kindly provided by William J. Welch. Quantification was performed using AIDA 3.26 image analysis software (Raytest, Straubenhardt, Germany).

### Example 8:

### Therapeutic application of novel potential antiprion-compounds identified by in vitro SIFT-assay in scrapie infected mice after day 100 post infection

In order to prove the effectiveness of those novel potential antiprion-compounds identified by the in vitro SIFT-screening assay and in particular of the N-Benzyliden-benzohydrazide (NBB) compound class in combating transmissible spongiform encephalopathies (TSE) or prion diseases an animal experiment was performed, in which the three compounds having the highest anti-prion activity in the scrapie cell culture assay were used to treat mice infected with the RML strain of scrapie at a very late stage of the incubation period. We choose the intraperitoneal application of the compounds at a 14 day time interval more than 100 days after infection, because this is typically the time when first subclinical symptoms appear in animals infected with this prion strain. This would correspond to the earliest time a TSE affected human being showing first symptoms of disease would realistically receive a therapeutic treatment. By choosing such very stringent conditions for therapy we wanted to assess the functionality of the tested substances as prion therapeutics in a realistic setting. Typically TSE therapeutics have to date only been tested for post-exposure prophylaxis in animal experiments, where they are applied around the time of infection with prions. In real life there are only very rare occasions, where such a therapeutic regime is feasible for TSE affected individuals. For most TSE patients the time of infection or begin of the incubation time for the familial and sporadic cases (which are the vast majority) is unknown and can not be realized. Therefore the majority of TSE patients will only be able to receive treatment after the occurrence of the first symptoms of TSE.

### Experimental procedures to Example 8:

### Scrapie-infection and treatment of mice

7-8 weeks-old, female C57BI6 mice were inoculated with RML scrapie by intracerebral injection of 30 µl of 10 % sterile brain homogenate in phosphate buffered saline (PBS) from mice terminally ill with the RML scrapie-strain. 102 days post infection, at a time, when first subclinical signs of scrapie disease (like head-tilt and fast rotation around the dorsal axis upon lifting the animal from the ground at its tail) were evident for some of the animals, these mice were treated with one selected novel potential antiprion-compound or with the vehicle, which was 75 % sterile, aqueous Dimethylsulfoxide (DMSO). Three potential antiprion-compounds had been selected according to their antiprion activity in the ScN2a cell culture model for this treatment, which were designated 293G02, 309F02, and 313B02 according to their plate-position in the Diverset chemical compound library (Chembridge Corp., San Diego, USA). The treatment with these compounds was carried out for 14 consecutive days by intraperitoneal injection of 50 µl per day of the respective compound dilution in the vehicle (75 % DMSO). Substances 293G02 and 309F02 were used at a concentration of 10 mM and 5 mM, respectively, throughout the entire period, while substance 313B02 was given initially also at 5 mM, but after the appearance of toxic signs, after day 4 the dose was reduced to 2.5 mM.

The animals were monitored daily for signs of disease by trained animal caretakers from day 80 post infection on. The animals were sacrificed, when they had reached the terminal stage of the disease marked by the presence of at least 3 out of 4 clinical symptoms, which are ataxia, tremor, difficulty in righting up from a position lying on its back, and tail stiffness. Typically the disease progress through the terminal stage of disease will lead to the death of the animal within one or two days. From the sacrificed animals one hemisphere and one half of the spleen were freshly frozen at -80 °C for western blot analysis, while the second hemisphere and the second half of the spleen as well as all inner organs were fixed in 4 % formaldehyde solution for (immune-)histology.

Occasionally some animals were found dead after they had only shown two clinical symptoms during the previous monitoring, presumably because the disease progress through the terminal stage was atypically fast in these cases. These corpses were frozen as whole animals at -80°C and brain and spleen of these animals were later extracted for western blot analysis only.

### Immunohistochemistry

Tissues were fixed in 4% formaldehyde solution for 2-3 days, treated for 1 h in 100 % formic acid, and fixed again for 2 days in 4 % formaldehyde solution. Tissue sections were paraffin embedded, cut into 4 µm slices, and transferred to slides. Slices were freed form paraffin by washing in a standard ethanol dilution row down to 0% ethanol in destilled water. Slides were pretreated by 30 min heating to 63 °C in 0.2 M boric acid at pH 9 and left to cool down for 15 min in the boric acid. After repeated washing in destilled water slices were equilibrated in PBS for two times 5 min.

Immunohistochemical staining of the tissue slices for PrP-Sc deposits was performed using a Benchmark staining automat (Ventana Inc., IIIkirch, France) running the program "BMK Enh V-Red Paraffin" and employing the "Enhanced Alkaline Phosphatase Red Detection Kit" (Ventana). The automatic staining procedure involves a Protease K (PK) digestion for 32 min, an incubation with the primary antibody for 32 min, and an incubation with the secondary antibody for 32 min. The PK digestion is carried out at PK concentration 91 µg/ml using, recombinant Proteinase K of PCR Grade (ROCHE, Penzberg, Germany)As primary antibody a polyclonal rabbit antibody against recombinant mouse PrP, designated CDC1 (Schmalzbauer, unpublished), was used at a dilution of 1:500 in Antibody-Diluent solution (Ventana). As secondary antibody a biotinylated, polyclonal pig-anti-rabbit IgG antibody, designated E 0353 (DAKOCytomation GmbH, Hamburg, Germany), was used as a 1:150 dilution in Antibody-Diluent solution (Ventana). Manual counterstaining of the slides was done by successive immersion in hemealaun solution for 1-2 min, tepide water for 10 min, the standard row of increasing ethanol concentration, and xylol before sealing of the slices with a cover glas.

### Results to Example 8

### Incubation time prolongation

As shown in Fig. 8 treatment of mice, which had been intracerebrally infected with the RML scrapie strain, at a late stage of the incubation time (days 102-115) by daily intraperitoneal application of 125-500 nmol of the three selected potential anti-prion drugs resulted in a prolongation of the incubation time, until the terminal stage of the scrapie infection was reached, for two of these drugs. The median of the survival time determined for substances 293G02 and 309F02 for groups of six animals each was prolonged by 9.5 and 11.5 days, respectively, in comparison to a group of eight animals that had received only the vehicle 75 % DMSO. For substance 313B02 no such prolongation of the median of the survival time in a group of seven treated animals compared to the same DMSO-control group could be found. To our knowledge this is the first case, where the administration of an antiprion drug so late in the incubation time, when first subclinical signs of disease have already manifested, has resulted in a measurable prolongation of the survival time of treated animals. The observed survival time prolongation correspond roughly to the duration of the treatment. This may imply that the treatment with these drugs has halted the disease progression as long as the drugs were administered. Though the disease has not been eradicated by the treatment, this result may indicate that it may be possible to attenuate the disease progress by a therapy using these substances. In this case the treatment with these drugs would lead to a prolongation of the lives of TSE-infected individuals and to a stabilization of their health status protecting it from further deterioration by halting the disease progress.

### PrP-Sc clearance in the spleen

Spleen tissue extracted after the death of drug-treated and vehicle-treated mice was immunohistochemically stained for the presence of PrP-Sc deposits. As shown in the example in Fig. 9 in the spleen of all five animals examined from the control-group PrP-Sc deposits were detectable, while in the spleen tissue from five of the animals, which had been treated with the substance 293G02, no signs of PrP-Sc deposition could be detected. This result may indicate an enhanced antiprion efficiency of this particular compound in peripheral tissues by the chosen experimental set-up and therapeutic regime. Since the application of the drug was intraperitoneally, it may have reached the spleen more easily than the brain of the infected animal. Therefore the therapy was not able to prevent PrP-Sc deposition and disease progression in the brain of the animal, presumably because the compound did not cross the blood-brain-barrier efficiently, but it was at least able to prevent or even revert the secondary infection of the spleen by prions spreading from the brain as the site of inoculation to the peripheral organs. Thus only pharmacokinetic investigations will clarify, whether there is a problem of bioavailablllty of this substance in the brain, that obscures the peripherally detectable effectiveness of this compound in preventing PrP-Sc accumulation and thereby spreading and progression of prion diseases.

### Example 9

### Effects of halogen substitutions on the anti-prion activity of N'-benzylidenebenzohydrazides

Figure 7A shows three structural classes characterized by the substitution of halogens (Fluorine, Chlorine, Bromine or lodine) at the benzyl ring of the benzylidene moiety at the N'-position of the NBB core. Interestingly, all three classes, defined by halogen substitutions in ortho-, meta-, or para-position (first, second, and third row, respectively) show similar SIFT activity distributions which are shifted towards positive values, indicating that these substitutions tend to increase the *in vitro* activity of the NBB motif.

While the structural classes of Figure 7A combine different halogen atom types, these atom types in the respective positions each define separate classes in Figure 7B. Regarding the ortho-position, the class of substances with a Fluorine substitution has a SIFT activity distribution whose median is only slightly larger than zero (Figure 7B, first row), while a Chlorine substitution (second row) or a Bromine substitution (third row) both shift the activity distribution noticeably towards higher anti-prion activity. Chlorine in ortho-position is found in numerous substances, about three quarters of which display positive SIFT activities. In contrast, there are only seven substances in which Bromine is substituted, five of them show positive SIFT activity.

Similarly Chlorine (fourth row) and Bromine (fifth row) substitutions in meta-position shift the SIFT activity distribution towards higher anti-prion activity as does Iodine substitution (sixth row), where the only two compounds containing this structural motif have clearly positive activities.

Also in the para-position Chlorine (eighth row) and Bromine (ninth row) substitutions are in most cases associated with positive SIFT-activities, while the distribution of the activities measured for the Fluorine substituted compounds centres around zero, indicating no activity in the average.

In conclusion this data indicates in general a positive effect of Chlorine, Bromine and lodine substitution of the benzylidene moiety on the anti-prion activity of NBB compounds, irrespective of the position of the substituent. In contrast Fluorine substitutions on average do not seem to have a positive effect on SIFT-activity, again irrespective of the position on the benzylidene ring.

### Example 10:

### Suppression of α-synuclein aggregation in vitro by N-benzyliden-benzohydrazide compound 293G02

Synucleinopathies are (neurodegenerative) diseases characterized by the intracellular accumulation of aggregates and fibrils composed mainly of the protein α-synuclein (for review see: Goedert, 2001). The most prominent neurodegenerative synucleinopathies are Parkinson's Disease (PD), Dementia with Lewy bodies (DLB), and multiple system atrophy (MSA).

The aggregation of α-synuclein *in vitro* has been demonstrated to occur in the presence of substances, such as alcohols, that mimic dielectric conditions as they exist naturally in the close vicinity of biological membranes (Munishkina et al., 2003). In particular low concentrations (8-12 %) of simple alcohols, such as ethanol, lead to a refolding of α-synuclein into a predominantly β-sheeted fold and a subsequent fibril formation by α-synuclein in vitro, thus representing a model system for the investigation of central aspects of α-synuclein misfolding and aggregation during the disease process.

In order to test selected N-Benzyliden-benzohydrazide (NBB) compounds for their potential to suppress α-synuclein aggregation we have used an in vitro system, where the organic solvent dimethylsulfoxid (DMSO) at low concentrations (0-3 %) was used instead of simple alcohols to induce α-synuclein aggregation in vitro. The multimer formation was monitored using a single particle fluorescence correlation set up by cross-correlation analysis and SIFT-analysis applied to mixtures of α-synuclein monomers labeled with green Alexa488- or red Alexa647-fluorophores, respectively. Such α-synuclein mixtures were aggregated in different DMSO concentrations in parallel to samples where an NBB compound was added to the reaction in one particular DMSO concentration.

### Experimental procedures to Example 10:

### Fluorescence labeling of α-synuclein

Recombinant α-synuclein was labeled with amino reactive fluorescent dyes, either with Alexa Fluor-488-O-succinimidyl ester or with Alexa Fluor-647-Orsuccinimidyl ester (Molecular Probes, USA), respectively, in reactions containing 100 mM NaHCO₃ at pH 9.5, 60 µM α-synuclein monomers and 120 µM of the respective fluorescent dye. The labeling reactions were carried out over night at 4 °C. After completion of the reaction unbound dye molecules were separated by size-exclusion chromatography of the reaction mixtures through two successive PD10 columns (Amersham Bioscience, Germany) preequilibated and eluted with 50 mM sodiumphosphate buffer of pH 7.0 according to the manufacturers instructions. The labeling efficiency and removal of unbound dye was determined by FCS measurements with suitable dilutions of fractions containing the labeled α-synuclein monomers. For the labeling ratio determination such measurements were compared to measurements of the same fractions predigested with proteinase K (0.1 mg/ ml) for 1 h at 37°C. The labeling ratios were 2.3 Alexa-488 dye molecules and 2.4 Alexa-647 dye molecules per α-synuclein monomer, respectively.

### Single particle fluorescence correlation measurements

In a 20 µl volume in the wells of a special micro-titer plate equipped with a glass cover slide bottom for fluorescence correlation measurements (Evotec-Technologies, Germany) α-synuclein aggregation was performed in a buffer containing 50 mM sodium phosphate at pH 7.0 and a mixture of α-synuclein monomers labeled with either Alexa488- or Alexa647-fluorophores, respectively, at a final concentration of approximately 5-10 nM of each α-synuclein species. For triplicate measurements three wells each further contained either, no DMSO, 1 % DMSO, or 1 % DMSO plus 10 µM compound 293G02, respectively. The measurements were started after addition of these alternative components and performed on an Insight fluorescence correlation instrument (Evotec-Technologies, Germany) using a 40x microscope objective of 1.2 NA (Olympus, Japan) with FIDA optical settings, a pinhole diameter of 70 µm, and 200 µW excitation with the 488 nm laser as well as 300 µW excitation with the 633 nm laser. Measurement time per well was 10 s, during which the laser focus was moved through the well by a beam scanner device using a scan path length of 100 µm at a scanning frequency of 50 Hz and a positioning table movement of 2000 µm. This is equivalent to a scanning speed of approximately 10 mm/s. After each measurement the laser focus was automatically and continuously moved to a neighbouring well in meanders covering the whole plate. Raw measurement data from meanders 19 to 25, which correspond to a period 3 to 5 hours after the start of the experiment and which represents the final plateau reached in α-synuclein aggregation kinetics in this experimental set-up, were used for mathematical fitting of the two-colour cross-correlation amplitude using the FCSPPEvalution software version 2.0 (Evoteo-OAI, Germany). Two-dimensional intensity distribution histograms, as in Fig. 11, were generated and analysed using the 2-D SIFT software (Evotec OAI, Germany).

### Results to Example 10:

### Suppression of α-synuclein aggregation by compound 293G02

The aggregation of α-synuclein caused by 1 % DMSO is reflected by an increase in the fluorescence cross-correlation, as seen in Fig. 10, in fluorescence correlation measurements 3-5 h after the addition of DMSO to the α-synuclein monomer solution. This increase is due to the formation of multimeric α-synuclein complexes that contain both red and green labeled α-synuclein units in higher numbers, as can be seen in Fig. 11. In contrast to the pure α-synuclein solution in **A,** where only very rarely more than 25 photons are detected during 40 µs from the molecules in the focus of the instrument laser in both colours simultaneously, the solution containing 1 % DMSO in **B** is characterized by the presence of a large number of complexes that emit up 50 photons in the red channel and up to 100 photons in the green channel during 40 µs bins. Although rarer, single bins can be found, where up to 150 photons in the red channel and up to 200 photons in the green channel have been counted.

The simultaneous addition of the NBB-compound 293G02 at a final concentration of 10 µM to the 1% DMSO solution is capable of inhibiting the formation of multimeric α-synuclein complexes to a large extent as can be seen in Fig. 10 from the low fluorescence cross-correlation, which is almost comparable to that of the measurement without DMSO and from Fig. 11 **C,** where the number of bins with more than 25 photons/bin in both colors is very small, again comparable to the situation without DMSO.

Thus compound 293G02 is able to inhibit efficiently the multimer formation of α-synuclein at a low micro molar concentration in this *in vitro* model for the pathological protein aggregation found in synucleinopathies. This is a clear indication that compound 293G02 can not only function as an anti-prion compound, but that it has also the potential to become a therapeutic compound for synucleinopathies, like Parkinson's Disease, DLB, and MSA, which interferes with the pathologic mechanism at the molecular level. Therefore compound 293G02 may represent the first candidate substance from the NBB class of chemicals with a proven capacity to inhibit α-synuclein aggregation in vitro, that will allow the development of a causative therapy against Parkinson's disease and other synucleinopathies.

Furthermore, the inhibitor activity of this compound on both, prion protein- and α-synuclein- aggregation in vitro, may reflect its general anti-aggregatory activity against a broader range of protein aggregation diseases, where protein misfolding into predominantly β-sheet conformations forms the basis for subsequent protein aggregation into amyloid fibrils. Therefore, this compound and probably further members of the NBB-class of substances may have the potential of being useful as therapeutics for the causative treatment of a whole panel of (neurodegenerative) protein aggregation diseases, ranging from transmissible spongiform encephalopathies, through Parkinson's disease, to Chorea Huntington or even to Alzheimer's disease and further to diabetes.

### References to Example 10:

Goedert, M., 2001, Alpha-synuclein and neurodegenerative diseases. Nat Rev Neuroscl. 2(7):492-501
Munishkina LA, Phelan C, Uversky VN, Fink AL., 2003, Conformational behavior and aggregation of alpha-synuclein in organic solvents: modeling the effects of membranes. Biochemistry 42(9):2720-30.

### References

Bach, S. et al. Isolation of drugs active against mammalian prions using a yeast-based screening assay. Nat Biotechnol 21, 1075-1081 (2003).
Bieschke, J. et al. Ultrasensitive detection of pathological prion protein aggregates by dual-color scanning for intensely fluorescent targets. 97, 5468-5473 (2000).
Borchelt, D.R., Taraboulos, A. & Prusiner, S.B. Evidence for synthesis of scrapie prion proteins in the endocytic pathway. J. Biol. Chem. 267, 16188-16199 (1992).
Borgelt, C. & Berthold, M.R. in IEEE International Conference on Data Mining (ICDM) 51-58 (IEEE Press, Piscataway, NJ, USA 2002, Maebashl, Japan; 2002).
Butler, D.A. et al. Scrapie-infected murine neuroblastoma cells produce protease-resistant prion proteins. J. Virol. 62, 1558-1564 (1988).
Caughey, B. & Raymond, G.J. The scrapie-associated form of PrP is made from a cell surface precursor that is both protease- and phospholipase-sensitive. J. Biol. Chem. 266, 18217-18223 (1991).
Caughey, W.S., Raymond, L.D., Horiuchi, M. & Caughey, B. Inhibition of protease-resistant prion protein formation by porphyrins and phthalocyanines. P Natl Acad Sci USA 95, 12117-12122 (1998).
Chabry, J., Caughey, B. & Chesebro, B. Specific inhibition of in vitro formation of protease-resistant prion protein by synthetic peptides. J Biol Chem 273, 13203-13207 (1998).
Demaimay, R., Chesebro, B. & Caughey, B. Inhibition of formation of protease-resistant prion protein by Trypan Blue, Sirius Red and other Congo Red analogs. 277-283 (2000).
Giese, A., Bieschke, J., Eigen, M. & Kretzschmar, H.A. Putting prions into focus: application of single molecule detection to the diagnosis of prion diseases. Arch Virol, 161-171 (2000).
Giese, A. & Kretzschmar, H.A. Prion-induced neuronal damage - The mechanisms of neuronal destruction in the subacute spongiform encephalopathies. Curr Top Microbiol 253, 203-217 (2001).
Gilch, S. et al. Intracellular re-routing of prion protein prevents propagation of PrPSc and delays onset of prion diseases. EMBO J 20, 3957-3966 (2001).
Horiuchi, M., Priola, S.A., Chabry, J. & Caughey, B. Interactions between heterologous forms of prion protein: Binding, inhibition of conversion, and species barriers. P Natl Acid Sci USA 97, 5836-5841 (2000).
Ihlenfeldt, W.D., Takahashi Y., Abe H. & S., S. Computation and Management of Chemical Properties in CACTVS: An Extensible Networked Approach toward Modularity and Compatibility. Journal of Chemical Information and Computer Sciences 34, 109-116 (1994).
Kiachopoulos, S., Heske, J., Tatzelt, J. & Winklhofer, K.F. Misfolding of the prion protein at the plasma membrane induces endocytosis, intracellular retention and degradation. Traffic in press (2004).
Kocisko, D.A. et al. New inhibitors of scrapie-associated prion protein formation in a library of 2,000 drugs and natural products (vol 77, pg 10288, 2003). J Virol 78, 3202-3202 (2004).
Kocisko, D.A. et al. New inhibitors of scrapie-associated prion protein formation in a library of 2,000 drugs and natural products. J Virol 77, 10288-10294 (2003).
Koltermann, A., Kettling, U., Bieschke, J., Winkler, T. & Eigen, M. Rapid assay processing by integration of dual-color fluorescence cross-correlation spectroscopy: High throughput screening for enzyme activity. P Nafl Acad Sci USA 95, 1421-1426 (1998).
Liemann, S. and R. Glockshuber (1999). "Influence of amino acid substitutions related to inherited human prion diseases on the thermodynamic stability of the cellular prion protein." Biochemistry 38(11): 3258-67.
Llewelyn, C.A. et al. Possible transmission of variant Creutzfeldt-Jakob disease by blood transfusion. Lancet 363, 417-421 (2004).
Mallucci, G. et al. Depleting neuronal PrP in prion infection prevents disease and reverses spongiosis. Science 302, 871-874 (2003).
Perrier, V. et al. Mimicking dominant negative inhibition of prion replication through structure-based drug design. P Natl Acad Sci USA 97, 6073-6078 (2000).
Post, K. et al. Rapid acquisition of beta-sheet structure in the prion protein prior to multimer formation. Biological Chemistry 379, 1307-1317 (1998).
Prusiner, S.B. Prions. P Natl Acad Sci USA 95, 13363-13383 (1998).
Rudyk, H. et al. Screening Congo Red and its analogues for their ability to prevent the formation of PrP-res in scrapie-infected cells. J Gen Virol 81, 1155-1164. (2000).
Safar J, Wille H, Itri V, Groth D, Serban H, Torchia M, Cohen FE, Prusiner SB.: Eight prion strains have PrP(Sc) molecules with different conformations. Nat Med. 1998 Oct;4(10):1157-65.
Schätzl, H.M. et al. A hypothalamic neuronal cell line persistently infected with scrapie prions exhibits apoptosis. J. Virol. 71, 8821-8831 (1997).
Schwille, P., Bieschke, J. & Oehlenschlager, F. Kinetic investigations by fluorescence correlation spectroscopy: The analytical and diagnostic potential of diffusion studies. Biophys Chem 66, 211-228 (1997).
Soto, C. et al. Reversion of prion protein conformational changes by synthetic beta-sheet breaker peptides. Lancet 355, 192-197 (2000).
Tatzelt, J., Prusiner, S.B. & Welch, W.J. Chemical chaperones interfere with the formation of scrapie prion protein. EMBO J 15, 6363-6373 (1996).
Vorberg I, Buschmann A, Harmeyer S, Saalmuller A, Pfaff E, Groschup MH. A novel epitope for the specific detection of exogenous prion proteins in transgenic mice and transfected murine cell lines. Virology. 1999 Mar 1;255(1):26-31
Will, R.G. et al. A new variant of Creutzfeldt-Jakob disease in the UK. Lancet 347, 921-925 (1996).Bruce, M.E. et al. Transmissions to mice indicate that 'new variant' CJD is caused by the BSE agent. Nature 389,498-501 (1997).
Winklhofer, K.F. & Tatzelt, J. Cationic lipopolyamines induce degradation of PrPSc in scrapie-infected mouse neuroblastoma cells. Biol Chem 381, 463-469 (2000).
Winklhofer, K.F., Hartl, F.U. & Tatzelt, J. A sensitive filter retention assay for the detection of PrPSc and the screening of anti-prion compounds. FEBS Lett. 503, 41-45 (2001).

## Claims

1. A method of identifying a compound for inhibiting aggregation of proteins involved in diseases linked to protein aggregation and/or neurodegenerative diseases, comprising the steps of:
(a) bringing into contact a labeled monomeric protein and a differently labeled aggregate of said protein in the (1) presence and (2) absence of a candidate inhibitor of aggregation which is selected from the group represented by 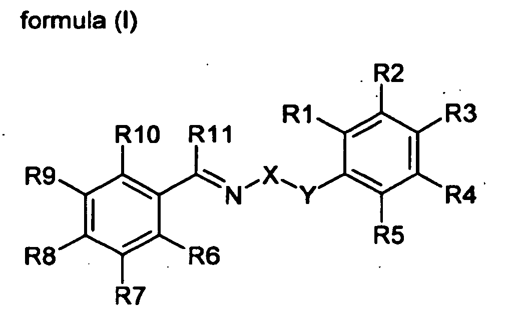 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R12 (if X is CH-R12), R13 (if X is N-R13) and R14 (if Y is CH-R14) are independently selected from hydrogen, alkyl, cycloalkyl, heterocycloalkyl, or hydroxyalkyl, each of which is optionally substituted;
(b) determining the amount of co-localized labels, representing the extent of binding of the monomeric proteins to the aggregates of said protein; and
(c) comparing the result obtained in the presence and absence of said compound,
wherein a decrease of co-localized labels in the presence of said compound is indicative of the compound's ability to inhibit aggregation of said protein.

2. The method of claim 1, wherein said labels are fluorescent labels.

3. The method of claim 1 or 2, wherein said label is attached to an antibody or a fragment of an antibody specifically bound to said protein.

4. The method of claim 3, wherein said antibody is capable of discriminating between the aggregated and monomeric protein.

5. The method of any one of claims 1 to 4, wherein the amount of co-localized labels is determined by using the method of "scanning for intensely fluorescent targets (SIFT)" or FRET or high resolution confocal imaging.

6. The method of any one of claims 1 to 5, wherein said monomeric and aggregating proteins are selected from the group consisting of prion protein, Amyloid precursor protein (APP), alpha-synuclein, superoxide dismutase, tau, immunoglobulin, Amyloid-A, transthyretin, Beta2-microglobulin, cystatin C, Apolipoproteine A1, Islet amyloid polypeptide, ANF, gelsolin, insulin, lysozyme, fibrinogen, huntingtin and ataxin and other proteins with a Poly-Q stretch, and fragments or derivates of said proteins.

7. The method of claim 6, wherein said monomeric protein is prion protein and said aggregated protein is PrP^{sc}.

8. The method of any one of claims 1 to 7, wherein said compound is selected from the group consisting of: 3,4-Dihydroxy-benzoic acid [1-naphthalen-2-yl-meth-(E)-ylidene]-hydrazide, 3-fluoro-N'-(3-bromo-4-methoxybenzylidene)-benzohydrazide, 3,4-dihydroxy-N'-(2,4-dichlorobenzylidene)-benzohydrazide, 2-Hydroxy-benzoic acid [1-naphthalen-2-yl-meth-(E)-ylidene]-hydrazide, N-(3,4-dimethoxy-phenylethyl)-3-nitro-benzylidene imine, 3-Chloro-benzoic acid [1-naphthalen-2-yl-meth-(E)-ylidene]-hydrazide and 2,4-Dihydroxy-benzoic acid [1-naphthalen-2-yl-meth-(E)-ylidene]-hydrazide.

9. The method of claim 8, wherein efficacy of said compound is further improved by derivatization.

10. A method of selecting compounds with in vivo efficacy in the treatment of diseases linked to protein aggregation and/or neurodegenerative diseases, comprising
(a) administering a candidate compound as defined in claim 1 to a cell culture or a non-human animal having the aggregatable isoform of the protein as defined in claim 6;
(b) quantifying the amount of observable aggregates; and
(c) identifying and selecting a compound which is capable of reducing aggregates or the formation of aggregates of said protein.

11. Use of a compound for inhibiting protein aggregation in vitro or ex vivo, wherein said compound is selected from the group represented by formula (I) 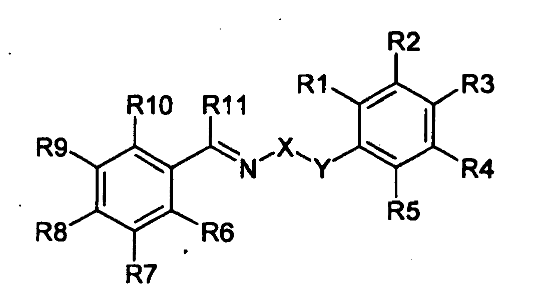 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R12 (if X is CH-R12), R13 (if X is N-R13) and R14 (if Y is CH-R14) are independently selected from hydrogen, alkyl, cycloalkyl, heterocycloalkyl, or hydroxyalkyl, each of which is optionally substituted.

12. The use of claim 10 or 11, wherein said compound is detectably labeled.

13. The use of any one of claims 10 to 12, wherein two or more of said compounds are used simultaneously.

14. Use of a compound for the preparation of a pharmaceutical composition for the treatment of a disease linked to protein aggregation and/or neurodegenerative disease, wherein said compound is selected from the group represented by formula (I) 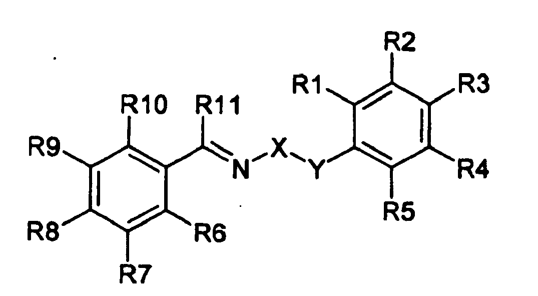 wherein in formula (I):
X is selected from CH-R12, and N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R12 (if X is CH-R12), R13 (if X is N-R13) and R14 (if Y is CH-R14) are independently selected from hydrogen, alkyl, cycloalkyl, heterocycloalkyl, or hydroxyalkyl, each of which is optionally substituted.

15. The use of claim 14, wherein said disease linked to protein aggregation is **characterized by** the presence of aggregated forms of at least one protein or a fragment or derivative thereof, wherein this protein is selected from the group consisting of prion protein, Amyloid precursor protein (APP), alpha-synuclein, superoxide dismutase, tau, immunoglobulin, Amyloid-A, transthyretin, Beta2-microglobulin, cystatin C, Apolipoproteine A1, Islet amyloid polypeptide, ANF, gelsolin, insulin, lysozyme, fibrinogen, huntingtin and ataxin and other proteins with a Poly-Q stretch.

16. The use of claim 14 or 15, wherein said disease is selected from the group consisting of Alzheimer's disease, prion disease, Parkinson's disease, multiple system atrophy, Diffuse Lewy body disease, frontotemporal dementia, amyotrophic lateral sclerosis, Huntington disease's, spinocerebellar ataxias and other Poly-Q diseases, hereditary cerebral amyloid angiopathy, familial amyloid polyneuropathy, primary systemic amyloidosis (AL amyloidosis), reactive systemic amyloidosis (AA amyloidosis), type II diabetes, injection-localized amyloidosis, beta-2 microglobulin amyloidosis, hereditary non-neuropathic amyloidosis, Finnish hereditary systemic amyloidosis.

17. The use of claim 16, wherein said prion disease is selected from Creutzfeldt-Jakob disease, variant Creutzfeldt-Jakob disease, genetic human prion disease, Bovine Spongiform Encephalopathy (BSE) and Scrapie.

18. The use of any one of claims 11 to 17, wherein said compound is selected from the group consisting of 3,4-Dihydroxy-benzoic acid [1-naphthalen-2-yl-meth-(E)-ylidene]-hydrazide, 3-fluoro-N'-(3-bromo-4-methoxybenzylidene)-benzohydrazide, 3,4-dihydroxy-N'-(2,4-dichlorobenzylidene)-benzohydrazide, 2-Hydroxy-benzoic acid [1-naphthalen-2-yl-meth-(E)-ylidene]-hydrazide, N-(3,4-dimethoxy-phenylethyl)-3-nitro-benzylidene imine, 3-Chloro-benzoic acid [1-naphthalen-2-yl-meth-(E)-ylidene]-hydrazide and 2,4-Dihydroxy-benzoic acid [1-naphthalen-2-yl-meth-(E)-ylidene]-hydrazide.

19. A pharmaceutical composition comprising a compound and optionally a pharmaceutically acceptable carrier or excipient, wherein said compound is selected from the group represented by formula (I) 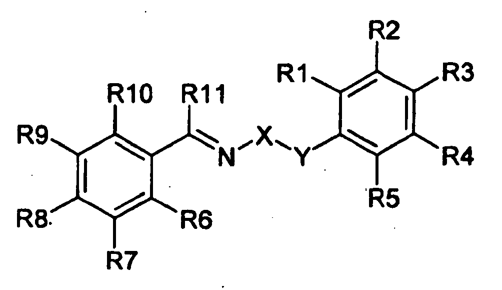 wherein in formula (I):
X is N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R13 (if X is N-R13) and R14 (if Y is CH-R14) are independently selected from hydrogen, alkyl, cycloalkyl, heterocycloalkyl, or hydroxyalkyl, each of which is optionally substituted.

20. A diagnostic composition comprising a compound selected from the group represented by formula (I) 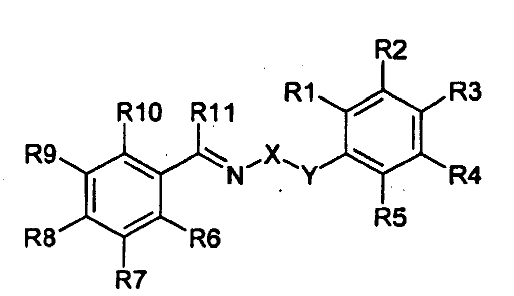 wherein in formula (I):
X is N-R13;
Y is selected from CH-R14, and C=O;
R1, R2, R3, R4, R5, R6, R7, R8, R9 and R10 are independently selected from hydrogen, halo, haloalkyl, aryl, fused aryl, carbocyclic, a heterocyclic group, a heteroaryl group, alkyl, alkenyl, alkynyl, arylalkyl, arylalkenyl, arylalkynyl, heteroarylalkyl, heteroarylalkenyl, heteroarylalkynyl, carbocycloalkyl, heterocycloalkyl, hydroxyalkyl, nitro, amino, cyano, acylamino, hydroxyl, thiol, sulfonyl, phosphonyl, acyloxy, azido, alkoxy, aryloxy, heteroaryloxy, arylalkoxy, heteroarylalkoxy, haloalkoxy, carboxy, carbonylamido and alkylthiol, each of which is optionally substituted;
R11, R13 (if X is N-R13) and R14 (if Y is CH-R14) are independently selected from hydrogen, alkyl, cycloalkyl, heterocycloalkyl, or hydroxyalkyl, each of which is optionally substituted.

21. The diagnostic composition of claim 20, wherein said compound is detectable or detectably labelled.

22. The composition of any one of claims 19 to 21, wherein said compound is selected from the group consisting of 3,4-Dihydroxy-benzoic acid [1-naphthalen-2-yl-meth-(E)-ylidene]-hydrazide, 3-fluoro-N'-(3-bromo-4-methoxybenzylidene)-benzohydrazide, 3,4-dihydroxy-N'-(2,4-dichlorobenzylidene)-benzohydrazide, 2-Hydroxy-benzoic acid [1-naphthalen-2-yl-meth-(E)-ylidene]-hydrazide, 3-Chloro-benzoic acid [1-naphthalen-2-yl-meth-(E)-ylidene]-hydrazide and 2,4-Dihydroxy-benzoic acid [1-naphthalen-2-yl-meth-(E)-ylidene]-hydrazide.

23. A kit comprising the compound as defined in any one of claims 19 to 22 and, in addition, an antibody or antibody fragment specifically binding to said compound; and/or monomeric or aggregated protein as defined in claim 6 or 7_{;} and/or monomeric or aggregated protein as defined in claim 6 or 7 optionally complexed with said compound; and instructions for use, in one or more container.

## Patentansprüche

1. Verfahren zur Identifizierung einer Verbindung zur Hemmung der Aggregation von Proteinen, die an Krankheiten, die mit Proteinaggregation in Zusammenhang stehen, und/oder neurodegenerativen Erkrankungen beteiligt sind, umfassend die Schritte:
(a) Inkontaktbringen eines markierten monomeren Proteins und eines unterschiedlich markierten Aggregats des Proteins in der (1) Anwesenheit und (2) Abwesenheit eines Inhibitorkandidaten der Aggregation, der ausgewählt ist aus der Gruppe dargestellt durch Formel (I) 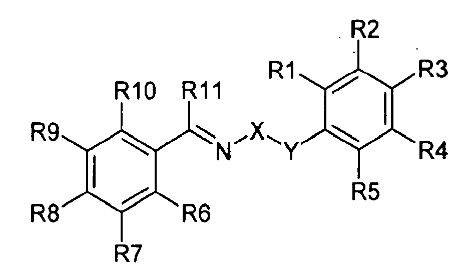 wobei in Formel (I):
X ausgewählt ist aus CH-R12 und N-R13;
Y ausgewählt ist aus CH-R14 und C=O;
R1, R2, R3, R4, R5, R6, R7, R8, R9 und R10 unabhängig ausgewählt sind aus Wasserstoff, Halogen, Haloalkyl, Aryl, kondensiertem Aryl, Carbocyclus, einem heterocyclischen Rest, einem Heteroarylrest, Alkyl, Alkenyl, Alkinyl, Arylalkyl, Arylalkenyl, Arylalkinyl, Heteroarylalkyl, Heteroarylalkenyl, Heteroarylalkinyl, Carbocycloalkyl, Heterocycloalkyl, Hydroxyalkyl, Nitro, Amino, Cyano, Acylamino, Hydroxyl, Thiol, Sulfonyl, Phosphonyl, Acyloxy, Azido, Alkoxy, Aryloxy, Heteroaryloxy, Arylalkoxy, Heteroarylalkoxy, Haloalkoxy, Carboxy, Carbonylamido und Alkylthiol, von denen jedes gegebenenfalls substituiert ist;
R11, R12 (wenn X CH-R12 ist), R13 (wenn X N-R13 ist) und R14 (wenn Y CH-R14 ist) unabhängig ausgewählt sind aus Wasserstoff, Alkyl, Cycloalkyl, Heterocycloalkyl oder Hydroxyalkyl, von denen jedes gegebenenfalls substituiert ist;
(b) Bestimmen der Menge an co-lokalisierten Markern, die das Ausmaß der Bindung der monomeren Proteine an die Aggregate des Proteins darstellt; und
(c) Vergleichen der in der Anwesenheit und Abwesenheit der Verbindung erhaltenen Ergebnisse,
wobei eine Verringerung der co-lokalisierten Marker in der Anwesenheit der Verbindung die Fähigkeit der Verbindung anzeigt, die Aggregation des Proteins zu hemmen.

2. Verfahren nach Anspruch 1, wobei die Marker fluoreszierende Marker sind.

3. Verfahren nach Anspruch 1 oder 2, wobei der Marker an einen Antikörper oder ein Fragment eines Antikörpers, der spezifisch an das Protein gebunden ist, angehängt ist.

4. Verfahren nach Anspruch 3, wobei der Antikörper in der Lage ist, zwischen dem aggregierten und dem monomeren Protein zu unterscheiden.

5. Verfahren nach einem der Ansprüche 1 bis 4, wobei die Menge der co-lokalisierten Marker durch die Verwendung der Methode des "scanning for intensely fluorescent targets (SIFT)" oder FRET oder hochauflösende konfokale Bildgebung bestimmt wird.

6. Verfahren nach einem der Ansprüche 1 bis 5, wobei die monomeren und aggregierten Proteine ausgewählt sind aus der Gruppe bestehend aus Prionprotein, Amyloid-Vorläuferprotein (APP), Alpha-Synuklein, Superoxiddismutase, Tau, Immunglobulin, Amyloid-A, Transthyretin, Beta2-Microglobulin, Cystatin C, Apolipoprotein A1, Islet-Amyloid-Polypeptid, ANF, Gelsolin, Insulin, Lysozym, Fibrinogen, Huntingtin und Ataxin und anderen Proteinen mit einem Poly-Q-Abschnitt, und Fragmenten oder Derivaten dieser Proteine.

7. Verfahren nach Anspruch 6, wobei das monomere Protein ein Prionprotein ist und das aggregierte Protein PrP^{SC} ist.

8. Verfahren nach einem der Ansprüche 1 bis 7, wobei die Verbindung ausgewählt ist aus der Gruppe bestehend aus: 3,4-Dihydroxybenzoesäure-[1-naphthalin-2-yl-meth-(E)-yliden]-hydrazid, 3-Fluor-N'-(3-Brom-4-methoxybenzyliden)-benzohydrazid, 3,4-Dihydroxy-N'-(2,4-dichlorbenzyliden)-benzohydrazid, 2-Hydroxybenzoesäure-[1-naphthalin-2-yl-meth-(E)-yliden]-hydrazid, N-(3,4-Dimethoxy-phenylethyl)-3-nitro-benzylidenimin, 3-Chlorbenzoesäure-[1-naphthalin-2-yl-meth-(E)-yliden]-hydrazid und 2,4-Dihydroxybenzoesäure-[1-naphthalin-2-yl-meth-(E)-yliden]-hydrazid.

9. Verfahren nach Anspruch 8, wobei die Wirksamkeit der Verbindung durch Derivatisierung weiter verbessert wird.

10. Verfahren zur Auswahl von Verbindungen mit in vivo-Wirksamkeit in der Behandlung von Krankheiten, die mit Proteinaggregation im Zusammenhang stehen, und/oder neurodegenerativen Erkrankungen, umfassend
(a) Verabreichen einer in Anspruch 1 definierten Kandidatenverbindung an eine Zellkultur oder ein nicht-menschliches Tier, das die aggregierbare Isoform des in Anspruch 6 definierten Proteins hat;
(b) Quantifizieren der Menge der beobachtbaren Aggregate; und
(c) Identifizieren und Auswählen einer Verbindung, die in der Lage ist, Aggregate oder die Bildung von Aggregaten des Proteins zu reduzieren.

11. Verwendung einer Verbindung zur Hemmung von Proteinaggregation in vitro oder ex vivo, wobei die Verbindung ausgewählt ist aus der Gruppe dargestellt durch Formel (I) 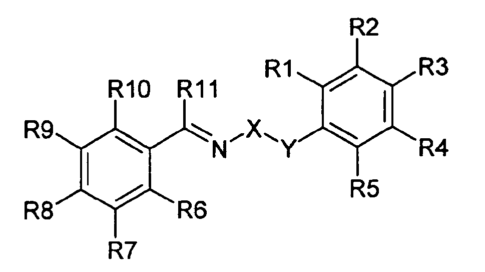 wobei in Formel (I):
X ausgewählt ist aus CH-R12 und N-R13;
Y ausgewählt ist aus CH-R14 und C=O;
R1, R2, R3, R4, R5, R6, R7, R8, R9 und R10 unabhängig ausgewählt sind aus Wasserstoff, Halogen, Haloalkyl, Aryl, kondensiertem Aryl, Carbocyclus, einem heterocyclischen Rest, einem Heteroarylrest, Alkyl, Alkenyl, Alkinyl, Arylalkyl, Arylalkenyl, Arylalkinyl, Heteroarylalkyl, Heteroarylalkenyl, Heteroarylalkinyl, Carbocycloalkyl, Heterocycloalkyl, Hydroxyalkyl, Nitro, Amino, Cyano, Acylamino, Hydroxyl, Thiol, Sulfonyl, Phosphonyl, Acyloxy, Azido, Alkoxy, Aryloxy, Heteroaryloxy, Arylalkoxy, Heteroarylalkoxy, Haloalkoxy, Carboxy, Carbonylamido und Alkylthiol, von denen jedes gegebenenfalls substituiert ist;
R11, R12 (wenn X CH-R12 ist), R13 (wenn X N-R13 ist) und R14 (wenn Y CH-R14 ist) unabhängig ausgewählt sind aus Wasserstoff, Alkyl, Cycloalkyl, Heterocycloalkyl, oder Hydroxyalkyl, von denen jedes gegebenenfalls substituiert ist.

12. Verwendung nach Anspruch 10 oder 11, wobei die Verbindung nachweisbar markiert ist.

13. Verwendung nach einem der Ansprüche 10 bis 12, wobei zwei oder mehr der Verbindungen gleichzeitig verwendet werden.

14. Verwendung einer Verbindung für die Herstellung einer pharmazeutischen Zusammensetzung zur Behandlung einer Krankheit, die mit Proteinaggregation in Zusammenhang steht, und/oder einer neurodegenerativen Erkrankung, wobei die Verbindung ausgewählt ist aus der Gruppe dargestellt durch Formel (I) 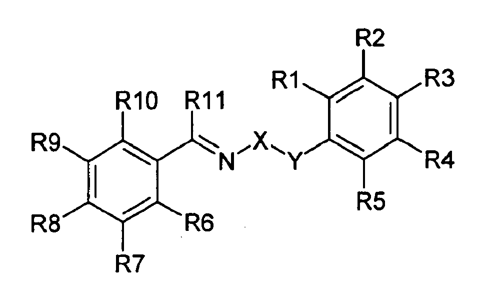 wobei in Formel (I):
X ausgewählt ist aus CH-R12 und N-R13;
Y ausgewählt ist aus CH-R14 und C=O;
R1, R2, R3, R4, R5, R6, R7, R8, R9 und R10 unabhängig ausgewählt sind aus Wasserstoff, Halogen, Haloalkyl, Aryl, kondensiertem Aryl, Carbocyclus, einem heterocyclischen Rest, einem Heteroarylrest, Alkyl, Alkenyl, Alkinyl, Arylalkyl, Arylalkenyl, Arylalkinyl, Heteroarylalkyl, Heteroarylalkenyl, Heteroarylalkinyl, Carbocycloalkyl, Heterocycloalkyl, Hydroxyalkyl, Nitro, Amino, Cyano, Acylamino, Hydroxyl, Thiol, Sulfonyl, Phosphonyl, Acyloxy, Azido, Alkoxy, Aryloxy, Heteroaryloxy, Arylalkoxy, Heteroarylalkoxy, Haloalkoxy, Carboxy, Carbonylamido und Alkylthiol, von denen jedes gegebenenfalls substituiert ist;
R11, R12 (wenn X CH-R12 ist), R13 (wenn X N-R13 ist) und R14 (wenn Y CH-R14 ist) unabhängig ausgewählt sind aus Wasserstoff, Alkyl, Cycloalkyl, Heterocycloalkyl oder Hydroxyalkyl, von denen jedes gegebenenfalls substituiert ist.

15. Verwendung nach Anspruch 14, wobei die mit Proteinaggregation in Zusammenhang stehende Krankheit durch die Anwesenheit aggregierter Formen wenigstens eines Proteins oder eines Fragments oder Derivats davon charakterisiert ist, wobei das Protein ausgewählt ist aus der Gruppe bestehend aus Prionprotein, Amyloid-Vorläuferprotein (APP), Alpha-Synuklein, Superoxiddismutase, Tau, Immunglobulin, Amyloid-A, Transthyretin, Beta2-Microglobulin, Cystatin C, Apolipoprotein A1, Islet-Amyloid-Polypeptid, ANF, Gelsolin, Insulin, Lysozym, Fibrinogen, Huntingtin und Ataxin und anderen Proteinen mit einem Poly-Q-Abschnitt.

16. Verwendung nach Anspruch 14 oder 15, wobei die Krankheit ausgewählt ist aus der Gruppe bestehend aus Alzheimer-Krankheit, Prionenkrankheit, Parkinson-Krankheit, multipler Systematrophie, diffuse Lewy-Korpuskel-Krankheit, frontotemporaler Demenz, amyotropher Lateralsklerose, Huntington-Krankheit, spinocerebellären Ataxien und anderen Poly-Q-Krankheiten, erblicher cerebraler amyloider Angiopathie, familiärer amyloider Polyneurophathie, primärer systemischer Amyloidose (AL-Amyloidose), reaktiver systemischer Amyloidose (AA-Amyloidose), Diabetes Typ II, durch Injektion lokalisierter Amyloidose, beta-2-Microglobulin-Amyloidose, erblicher nicht-neuropathischer Amyloidose, Finnischer erblicher systemischer Amyloidose.

17. Verwendung nach Anspruch 16, wobei die Prionenkrankheit ausgewählt ist aus Creutzfeldt-Jakob-Krankheit, Creutzfeldt-Jakob-Krankheit-Variante, genetischer menschlicher Prionenkrankheit, boviner spongiformer Encephalopathie (BSE) und Traberkrankheit.

18. Verwendung nach einem der Ansprüche 11 bis 17, wobei die Verbindung ausgewählt ist aus der Gruppe bestehend aus 3,4-Dihydroxybenzoesäure-[1-naphthalin-2-yl-meth-(E)-yliden]-hydrazid, 3-Fluor-N'-(3-brom-4-methoxybenzyliden)-benzohydrazid, 3,4-Dihydroxy-N'-(2,4-dichlorbenzyliden)-benzohydrazid, 2-Hydroxybenzoesäure-[1-naphthalin-2-yl-meth-(E)-yliden]-hydrazid, N-(3,4-Dimethoxy-phenylethyl)-3-nitro-benzylidenimin, 3-Chlorbenzoesäure-[1-naphthalin-2-yl-meth-(E)-yliden]-hydrazid und 2,4-Dihydroxybenzoesäure [1-naphthalin-2-yl-meth-(E)-yliden]-hydrazid.

19. Pharmazeutische Zusammensetzung, umfassend eine Verbindung und gegebenenfalls einen pharmazeutisch verträglichen Träger oder Hilfsstoff, wobei die Verbindung ausgewählt ist aus der Gruppe dargestellt durch Formel (I) 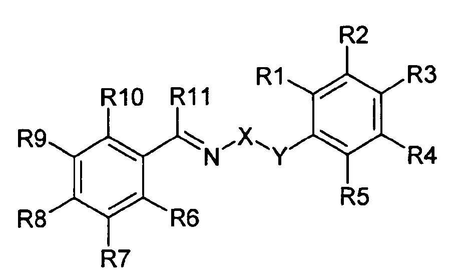 wobei in Formel (I):
X N-R13 ist;
Y ausgewählt ist aus CH-R14 und C=O;
R1, R2, R3, R4, R5, R6, R7, R8, R9 und R10 unabhängig ausgewählt sind aus Wasserstoff, Halogen, Haloalkyl, Aryl, kondensiertem Aryl, Carbocyclus, einem heterocyclischen Rest, einem Heteroarylrest, Alkyl, Alkenyl, Alkinyl, Arylalkyl, Arylalkenyl, Arylalkinyl, Heteroarylalkyl, Heteroarylalkenyl, Heteroarylalkinyl, Carbocycloalkyl, Heterocycloalkyl, Hydroxyalkyl, Nitro, Amino, Cyano, Acylamino, Hydroxyl, Thiol, Sulfonyl, Phosphonyl, Acyloxy, Azido, Alkoxy, Aryloxy, Heteroaryloxy, Arylalkoxy, Heteroarylalkoxy, Haloalkoxy, Carboxy, Carbonylamido und Alkylthiol, von denen jedes gegebenenfalls substituiert ist;
R11, R13 (wenn X N-R13 ist) und R14 (wenn Y CH-R14 ist) unabhängig ausgewählt sind aus Wasserstoff, Alkyl, Cycloalkyl, Heterocycloalkyl oder Hydroxyalkyl, von denen jedes gegebenenfalls substituiert ist.

20. Diagnostische Zusammensetzung, umfassend eine Verbindung ausgewählt aus der Gruppe dargestellt durch Formel (I) 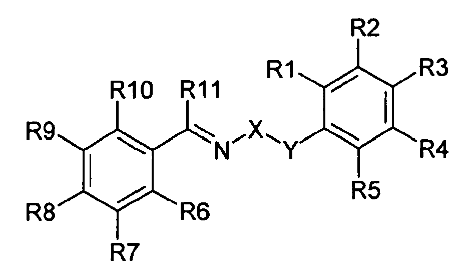 wobei in Formel (I):
X N-R13 ist;
Y ausgewählt ist aus CH-R14 und C=O;
R1, R2, R3, R4, R5, R6, R7, R8, R9 und R10 unabhängig ausgewählt sind aus Wasserstoff, Halogen, Haloalkyl, Aryl, kondensiertem Aryl, Carbocyclus, einem heterocyclischen Rest, einem Heteroarylrest, Alkyl, Alkenyl, Alkinyl, Arylalkyl, Arylalkenyl, Arylalkinyl, Heteroarylalkyl, Heteroarylalkenyl, Heteroarylalkinyl, Carbocycloalkyl, Heterocycloalkyl, Hydroxyalkyl, Nitro, Amino, Cyano, Acylamino, Hydroxyl, Thiol, Sulfonyl, Phosphonyl, Acyloxy, Azido, Alkoxy, Aryloxy, Heteroaryloxy, Arylalkoxy, Heteroarylalkoxy, Haloalkoxy, Carboxy, Carbonylamido und Alkylthiol, von denen jedes gegebenenfalls substituiert ist; R11, R13 (wenn X N-R13 ist) und R14 (wenn Y CH-R14 ist) unabhängig ausgewählt sind aus Wasserstoff, Alkyl, Cycloalkyl, Heterocycloalkyl oder Hydroxyalkyl, von denen jedes gegebenenfalls substituiert ist.

21. Diagnostische Zusammensetzung nach Anspruch 20, wobei die Verbindung nachweisbar ist oder nachweisbar markiert ist.

22. Zusammensetzung nach einem der Ansprüche 19 bis 21, wobei die Verbindung ausgewählt ist aus der Gruppe bestehend aus 3,4-Dihydroxybenzoesäure-[1-naphthalin-2-yl-meth-(E)-yliden]-hydrazid, 3-Fluor-N'-(3-brom-4-methoxybenzyliden)-benzohydrazid, 3,4-Dihydroxy-N'-(2,4-dichlorbenzyliden)-benzohydrazid, 2-Hydroxybenzoesäure-[1-naphthalin-2-yl-meth-(E)-yliden]-hydrazid, 3-Chlorbenzoesäure-[1-naphthalin-2-yl-meth-(E)-yliden]-hydrazid und 2,4- Dihydroxybenzoesäure-[1-naphthalin-2-yl-meth-(E)-yliden]-hydrazid.

23. Kit, umfassend die Verbindung wie in einem der Ansprüche 19 bis 22 definiert und zusätzlich einen Antikörper oder ein Antikörperfragment, der/das spezifisch an die Verbindung bindet; und/oder monomeres oder aggregiertes Protein wie in Anspruch 6 oder 7 definiert; und/oder monomeres oder aggregiertes Protein wie in Anspruch 6 oder 7 definiert gegebenenfalls im Komplex mit der Verbindung; und Anweisungen zur Verwendung, in einem oder mehreren Behältern.

## Revendications

1. Procédé d'identification d'un composé permettant d'inhiber l'agrégation des protéines impliquées dans les maladies liées à l'agrégation des protéines et/ou les maladies neurodégénératives, comprenant les étapes consistant à :
(a) mettre en contact une protéine monomère marquée et un agrégat de ladite protéine marqué différemment (1) en présence et (2) en l'absence d'un candidat inhibiteur de l'agrégation, qui est choisi parmi le groupe représenté par 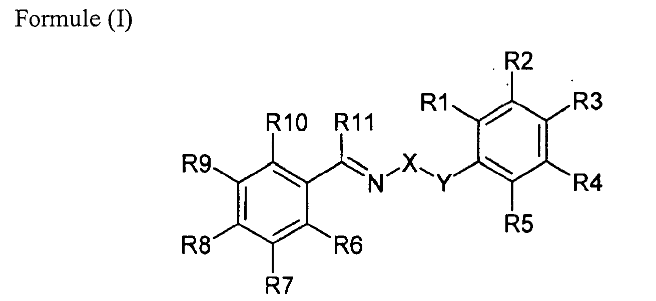 où, dans la formule (I) :
X est choisi parmi CH-R12 et N-R13 ;
Y est choisi parmi CH-R14 et C=O ;
R1, R2, R3, R4, R5, R6, R7, R8, R9 et R10 sont indépendamment choisis parmi l'hydrogène, un halogène, un haloalkyle, un aryle, un aryle fusionné, un carbocyclique, un groupe hétérocyclique, un groupe hétéroaryle, un alkyle, un alcényle, un alcynyle, un arylaklyle, un arylalcényle, un arylalcynyle, un hétéroarylalkyle, un hétéroarylalcényle, un hétéroarylalcynyle, un carbocycloalkyle, un hétérocycloalkyle, un hydroxyalkyle, un nitro, un amino, un cyano, un acylamino, un hydroxyle, un thiol, un sulfonyle, un phosphonyle, un acyloxy, un azido, un alcoxy, un aryloxy, un hétéroaryloxy, un arylalcoxy, un hétéroarylalcoxy, un haloalcoxy, un carboxy, un carbonylamido, et un alkylthio, dont chacun est éventuellement substitué ;
R11, R12 (si X est CH-R12), R13 (si X est N-R13) et R14 (si Y est CH-R14) sont indépendamment choisis parmi l'hydrogène, un alkyle, un cycloalkyle, un hétérocycloalkyle, ou un hydroxyalkyle, dont chacun est éventuellement substitué ;
(b) déterminer la quantité de marqueurs co-localisés, représentant l'étendue de liaison des protéines monomèriques aux agrégats de ladite protéine ; et
(c) comparer les résultats obtenus en présence et en l'absence dudit composé,
dans lequel une diminution des marqueurs co-localisés en présence dudit composé indique la capacité du composé à inhiber l'agrégation de ladite protéine.

2. Procédé selon la revendication 1, dans lequel les marqueurs sont des marqueurs fluorescents.

3. Procédé selon la revendication 1 ou 2, dans lequel ledit marqueur est fixé sur un anticorps ou sur un fragment d'anticorps spécifiquement lié à ladite protéine.

4. Procédé selon la revendication 3, dans lequel ledit anticorps est capable de discriminer entre les protéines agrégées et les protéines monomèriques.

5. Procédé selon l'une quelconque des revendications 1 à 4, dans lequel la quantité de marqueurs co-localisés est déterminée en utilisant le procédé appelé « balayage des cibles intensément fluorescentes » (SIFT) ou FRET ou par imagerie confocale haute résolution.

6. Procédé selon l'une quelconque des revendications 1 à 5, dans lequel lesdites protéines monomèriques et agrégées sont choisies parmi le groupe constitué par la protéine de prion, le précurseur de la protéine amyloïde (APP), l'alpha-synucléine, la superoxyde dismutase, tau, une immunoglobuline, l'amyloïde-A, la transthyrétine, la bêta2-microglobuline, la cystatine C, l'apolipoprotéine A1, l'amyline, l'ANF, la gelsoline, l'insuline, le lysozyme, le fibrinogène, l'huntingtine et l'ataxine, et d'autres protéines avec une extension poly-Q, et des fragments ou dérivés desdites protéines.

7. Procédé selon la revendication 6, dans lequel ladite protéine monomèrique est une protéine de prion et ladite protéine agrégée est PrP^{SC}.

8. Procédé selon l'une quelconque des revendications 1 à 7, dans lequel ledit composé est choisi parmi le groupe constitué par : le [1-naphtalèn-2-yl-méth-(E)-ylidène]-hydrazide d'acide 3,4-dihydroxy-benzoïque, le 3-fluoro-N'-(3-bromo-4-méthoxybenzylidène)-benzohydrazide, le 3,4-dihydroxy-N'-(2,4-dichlorobenzylidène)-benzohydrazide, le [1-naphtalèn-2-yl-méth-(E)-ylidène]-hydrazide d'acide 2-hydroxy-benzoïque, le N-(3,4-diméthoxy-phényléthyl)-3-nitro-benzylidène imine, le [1-naphtalèn-2-yl-méth-(E)-ylidène]-hydrazide d'acide 3-chloro-benzoïque et le [1-naphtalèn-2-yl-méth-(E)-ylidène]-hydrazide d'acide 2,4-dihydroxy-benzoïque.

9. Procédé selon la revendication 8, dans lequel l'efficacité dudit composé est encore améliorée par dérivation.

10. Procédé de sélection de composés ayant une efficacité *in vivo* dans le traitement des maladies liées à l'agrégation de protéines et/ou des maladies neurodégénératives, comprenant les étapes consistant à :
(a) administrer un composé candidat tel que défini dans la revendication 1 à une culture cellulaire ou à un animal non humain ayant l'isoforme agrégeable de la protéine telle que définie dans la revendication 6 ;
(b) quantifier la quantité d'agrégats observables ; et
(c) identifier et choisir un composé qui soit capable de réduire les agrégats ou la formation d'agrégats de ladite protéine.

11. Utilisation d'un composé permettant d'inhiber l'agrégation de protéines *in vivo* ou *ex vivo*, dans laquelle ledit composé est choisi parmi le groupe représenté par la formule (I) 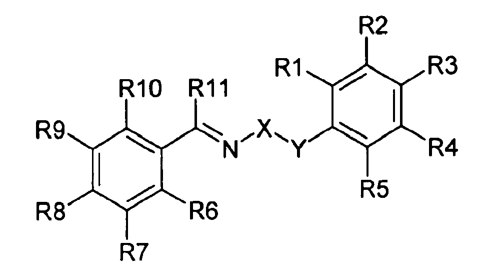 où, dans la formule (I) :
X est choisi parmi CH-R12 et N-R13 ;
Y est choisi parmi CH-R14 et C=O ;
R1, R2, R3, R4, R5, R6, R7, R8, R9 et R10 sont indépendamment choisis parmi l'hydrogène, un halogène, un haloalkyle, un aryle, un aryle fusionné, un carbocyclique, un groupe hétérocyclique, un groupe hétéroaryle, un alkyle, un alcényle, un alcynyle, un arylaklyle, un arylalcényle, un arylalcynyle, un hétéroarylalkyle, un hétéroarylalcényle, un hétéroarylalcynyle, un carbocycloalkyle, un hétérocycloalkyle, un hydroxyalkyle, un nitro, un amino, un cyano, un acylamino, un hydroxyle, un thiol, un sulfonyle, un phosphonyle, un acyloxy, un azido, un alcoxy, un aryloxy, un hétéroaryloxy, un arylalcoxy, un hétéroarylalcoxy, un haloalcoxy, un carboxy, un carbonylamido, et un alkylthio, dont chacun est éventuellement substitué ;
R11, R12 (si X est CH-R12), R13 (si X est N-R13) et R14 (si Y est CH-R14) sont indépendamment choisis parmi l'hydrogène, un alkyle, un cycloalkyle, un hétérocycloalkyle, ou un hydroxyalkyle, dont chacun est éventuellement substitué.

12. Utilisation selon la revendication 10 ou 11, dans laquelle ledit composé est marqué de façon détectable.

13. Utilisation selon l'une quelconque des revendications 10 à 12, dans laquelle deux ou plusieurs desdits composés sont utilisés simultanément.

14. Utilisation d'un composé pour la préparation d'une composition pharmaceutique pour le traitement d'une maladie liée à l'agrégation des protéines et/ou d'une maladie neurodégénérative, dans laquelle ledit composé est choisi parmi le groupe représenté par la formule (I) 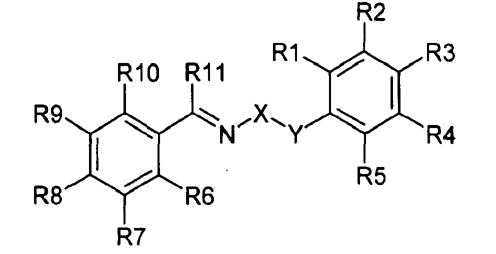 où, dans la formule (I) :
X est choisi parmi CH-R12 et N-R13 ;
Y est choisi parmi CH-R14 et C=O ;
R1, R2, R3, R4, R5, R6, R7, R8, R9 et R10 sont indépendamment choisis parmi l'hydrogène, un halogène, un haloalkyle, un aryle, un aryle fusionné, un carbocyclique, un groupe hétérocyclique, un groupe hétéroaryle, un alkyle, un alcényle, un alcynyle, un arylaklyle, un arylalcényle, un arylalcynyle, un hétéroarylalkyle, un hétéroarylalcényle, un hétéroarylalcynyle, un carbocycloalkyle, un hétérocycloalkyle, un hydroxyalkyle, un nitro, un amino, un cyano, un acylamino, un hydroxyle, un thiol, un sulfonyle, un phosphonyle, un acyloxy, un azido, un alcoxy, un aryloxy, un hétéroaryloxy, un arylalcoxy, un hétéroarylalcoxy, un haloalcoxy, un carboxy, un carbonylamido, et un alkylthio, dont chacun est éventuellement substitué ;
R11, R12 (si X est CH-R12), R13 (si X est N-R13) et R14 (si Y est CH-R14) sont indépendamment choisis parmi l'hydrogène, un alkyle, un cycloalkyle, un hétérocycloalkyle, ou un hydroxyalkyle, dont chacun est éventuellement substitué.

15. Utilisation selon la revendication 14, dans laquelle ladite maladie liée à l'agrégation de protéines est **caractérisée par** la présence de formes agrégées d'au moins une protéine ou un fragment ou dérivé de celle-ci, dans laquelle cette protéine est choisie parmi le groupe constitué par la protéine de prion, le précurseur de la protéine amyloïde (APP), l'alpha-synucléine, la superoxyde dismutase, tau, une immunoglobuline, l'amyloïde-A, la transthyrétine, la bêta2-microglobuline, la cystatine C, l'apolipoprotéine A1, l'amyline, l'ANF, la gelsoline, l'insuline, le lysozyme, le fibrinogène, l'huntingtine et l'ataxine, et d'autres protéines avec une extension poly-Q.

16. Utilisation selon la revendication 14 ou 15, dans laquelle ladite maladie est choisie parmi le groupe constitué par la maladie d'Alzheimer, la maladie du prion, la maladie de Parkinson, l'atrophie multi-systémique, la maladie à corpuscules de Lewis diffuse, la démence frontotemporale, la sclérose amyotrophique latérale, la maladie de Huntington, l'ataxie spinocérébelleuse, et d'autres maladies liées au Poly-Q, l'angiopathie amyloïde cérébrale héréditaire, la polyneuropathie amyloïde familiale, l'amyloïdose systémique primaire (amyloïdose AL), l'amyloïdose systémique réactive (amyloïdose AA), le diabète de type II, l'amyloïdose localisée par injection, l'amyloïdose de la microglobuline bêta-2, l'amyloïdose non-neuropathique héréditaire, l'amyloïdose sytémique héréditaire de Finlande.

17. Utilisation selon la revendication 16, dans laquelle ladite maladie du prion est choisie parmi la maladie de Creutzfeldt-Jakob, la variante de la maladie de Creutzfeldt-Jakob, la maladie du prion humaine génétique, l'encéphalopathie spongiforme bovine (ESB) et la tremblante du mouton.

18. Utilisation selon l'une quelconque des revendications 11 à 17, dans laquelle ledit composé est choisi parmi le groupe constitué par : le [1-naphtalèn-2-yl-méth-(E)-ylidène]-hydrazide d'acide 3,4-dihydroxy-benzoïque, le 3-fluoro-N'-(3-bromo-4-méthoxybenzylidène)-benzohydrazide, le 3,4-dihydroxy-N'-(2,4-dichlorobenzylidène)-benzohydrazide, le [1-naphtalèn-2-yl-méth-(E)-ylidène]-hydrazide d'acide 2-hydroxy-benzoïque, le N-(3,4-diméthoxy-phényléthyl)-3-nitro-benzylidène imine, le [1-naphtalèn-2-yl-méth-(E)-ylidène]-hydrazide d'acide 3-chloro-benzoïque et le [1-naphtalèn-2-yl-méth-(E)-ylidène]-hydrazide d'acide 2,4-dihydroxy-benzoïque.

19. Composition pharmaceutique comprenant un composé et éventuellement un support ou excipient acceptable sur le plan pharmaceutique, dans laquelle ledit composé est choisi parmi le groupe représenté par la formule (I) 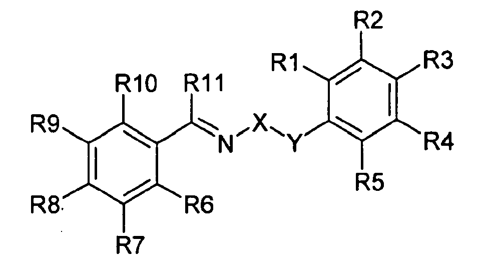 où, dans la formule (I) :
X est N-R13 ;
Y est choisi parmi CH-R14 et C=O ;
R1, R2, R3, R4, R5, R6, R7, R8, R9 et R10 sont indépendamment choisis parmi l'hydrogène, un halogène, un haloalkyle, un aryle, un aryle fusionné, un carbocyclique, un groupe hétérocyclique, un groupe hétéroaryle, un alkyle, un alcényle, un alcynyle, un arylaklyle, un arylalcényle, un arylalcynyle, un hétéroarylalkyle, un hétéroarylalcényle, un hétéroarylalcynyle, un carbocycloalkyle, un hétérocycloalkyle, un hydroxyalkyle, un nitro, un amino, un cyano, un acylamino, un hydroxyle, un thiol, un sulfonyle, un phosphonyle, un acyloxy, un azido, un alcoxy, un aryloxy, un hétéroaryloxy, un arylalcoxy, un hétéroarylalcoxy, un haloalcoxy, un carboxy, un carbonylamido, et un alkylthio, dont chacun est éventuellement substitué ;
R11, R13 (si X est N-R13) et R14 (si Y est CH-R14) sont indépendamment choisis parmi l'hydrogène, un alkyle, un cycloalkyle, un hétérocycloalkyle, ou un hydroxyalkyle, dont chacun est éventuellement substitué.

20. Composition de diagnostic comprenant un composé choisi parmi le groupe représenté par la formule (I) 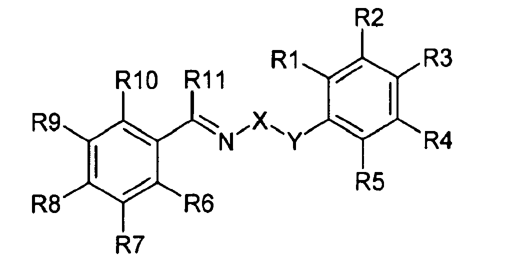 où, dans la formule (I) :
X est N-R13 ;
Y est choisi parmi CH-R14 et C=O ;
R1, R2, R3, R4, R5, R6, R7, R8, R9 et R10 sont indépendamment choisis parmi l'hydrogène, un halogène, un haloalkyle, un aryle, un aryle fusionné, un carbocyclique, un groupe hétérocyclique, un groupe hétéroaryle, un alkyle, un alcényle, un alcynyle, un arylaklyle, un arylalcényle, un arylalcynyle, un hétéroarylalkyle, un hétéroarylalcényle, un hétéroarylalcynyle, un carbocycloalkyle, un hétérocycloalkyle, un hydroxyalkyle, un nitro, un amino, un cyano, un acylamino, un hydroxyle, un thiol, un sulfonyle, un phosphonyle, un acyloxy, un azido, un alcoxy, un aryloxy, un hétéroaryloxy, un arylalcoxy, un hétéroarylalcoxy, un haloalcoxy, un carboxy, un carbonylamido, et un alkylthio, dont chacun est éventuellement substitué ;
R11, R13 (si X est N-R13) et R14 (si Y est CH-R14) sont indépendamment choisis parmi l'hydrogène, un alkyle, un cycloalkyle, un hétérocycloalkyle, ou un hydroxyalkyle, dont chacun est éventuellement substitué.

21. Composition de diagnostic selon la revendication 20, dans laquelle ledit composé est détectable ou marqué de façon détectable.

22. Composition selon l'une quelconque des revendications 19 à 21, dans laquelle ledit composé est choisi parmi le groupe constitué par le [1-naphtalèn-2-yl-méth-(E)-ylidène]-hydrazide d'acide 3,4-dihydroxy-benzoïque, le 3-fluoro-N'-(3-bromo-4-méthoxybenzylidène)-benzohydrazide, le 3,4-dihydroxy-N'-(2,4-dichlorobenzylidène)-benzohydrazide, le [1-naphtalèn-2-yl-méth-(E)-ylidène]-hydrazide d'acide 2-hydroxy-benzoïque, le [1-naphtalèn-2-yl-méth-(E)-ylidène]-hydrazide d'acide 3-chloro-benzoïque et le [1-naphtalèn-2-yl-méth-(E)-ylidène]-hydrazide d'acide 2,4-dihydroxy-benzoïque.

23. Kit comprenant le composé tel que défini dans l'une quelconque des revendications 19 à 22 et, en outre, un anticorps ou un fragment d'anticorps se liant spécifiquement audit composé ; et/ou une protéine monomèrique ou agrégée telle que définie dans la revendication 6 ou 7, éventuellement complexée avec ledit composé ; et des instructions d'utilisation, dans un ou plusieurs contenants.
